Europäisches Patentamt

European Patent Office

Office européen des brevets

(19)

(11)  EP 0 981 609 B1

(12)  **EUROPEAN PATENT SPECIFICATION**

(45) Date of publication and mention
of the grant of the patent:
**19.09.2001   Bulletin 2001/38**

(21) Application number: **98919092.1**

(22) Date of filing: **13.05.1998**

(51) Int Cl.[7]: **C12N 15/10**, C12Q 1/68

(86) International application number:
**PCT/DK98/00186**

(87) International publication number:
**WO 98/51789 (19.11.1998 Gazette 1998/46)**

(54) **A METHOD TO CLONE MRNAS AND DISPLAY OF DIFFERENTIALLY EXPRESSED TRANSCRIPTS (DODET)**

EINE METHODE ZUM KLONIEREN VON MRNA UND DARSTELLUNG VON DIFFERENTIELL EXPRIMIERTEN TRANSCRIPTEN (DODET)

METHODE PERMETTANT DE CLONER DES ARNm ET D'AFFICHER DES TRANSCRITS A EXPRESSION DIFFERENTIELLE (DODETS)

(84) Designated Contracting States:
**AT BE CH CY DE DK ES FI FR GB GR IE IT LI LU MC NL PT SE**

(30) Priority: **13.05.1997   DK 54797
19.05.1997   US 871030
27.03.1998   DK 43298**

(43) Date of publication of application:
**01.03.2000   Bulletin 2000/09**

(60) Divisional application:
**01202318.0**

(73) Proprietor: **Azign Bioscience A/S
2450 Copenhagen SV (DK)**

(72) Inventor: **WARTHOE, Peter, Rolf
DK-2100 Copenhagen (DK)**

(74) Representative:
**Plougmann, Vingtoft & Partners A/S
Sankt Annae Plads 11,
P.O. Box 3007
1021 Copenhagen K (DK)**

(56) References cited:
EP-A- 0 743 367          WO-A-93/18176
WO-A-95/08647          WO-A-95/13369
WO-A-97/05286          WO-A-97/29211

• BAUER D ET AL: "DIFFERENTIAL DISPLAY RESERSE TRANSCRIPTION PCR" BIOTEC, no. 2, April 1994, page 32, 34/35 XP000198999
• R. WILLIAMSON: "The preparation and screening of a cDNA clone bank" GENETIC ENGINEERING 1 , ED. R. WILLIAMSON, ACADEMIC PRESS, INC. ,1981, pages 1-59, XP002076424 New York, US
• "Construction and analysis of cDNA libraries" MOLECULAR CLONING, SECOND EDITION, J. SAMBROOK, E.F. FRITSCH AND T. MANIATIS, , 1989, pages 8.11-8.17, XP002076425 CSH, US
• PATANJALI S R ET AL: "CONSTRUCTION OF A UNIFORM-ABUNDANCE (NORMALIZED) CDNA LIBRARY" PROCEEDINGS OF THE NATIONAL ACADEMY OF SCIENCES OF USA, vol. 88, no. 5, 1 March 1991, pages 1943-1947, XP000368687

**Description**

BACKGROUND OF THE INVENTION

The human body is comprised primarily of specialised cells performing different physiological functions organised into organs and tissues. All human cells contain DNA, arranged in a series of sub-units known as genes. It is estimated that there are approximately 100,000 genes in the human genome. Genes are the blueprints for proteins. Proteins may perform a wide variety of biological functions, for example messengers, catalysts and sensors. Such compounds are responsible for managing most of the physiological and biochemical functions in humans and all other living organisms. Over the last few decades, there has been a growing recognition that many major diseases have a genetic basis. It is now well established that genes play an important role in cancer, cardiovascular diseases, psychiatric disorders, obesity, and metabolic diseases. Significant resources are being focused on genomic research based on the notion that the nucleotide sequences of a particular gene and its predicted protein product will lead to an understanding of its function in healthy and malfunctioning cells or tissues. This understanding is expected, in turn, to lead to therapeutic and diagnostic approaches, focused on molecular targets associated with the gene and the protein it expresses. The first step on the way to the development of such applications is to identify the genes specifically involved in the different categories of diseases. Application of this knowledge can produce new and valuable markers, identifying regions producing major diseases to be used for diagnostic and therapeutic benefit.

Faced with the high complexity of the human genome, many approaches are being used to unravel the connection between primary gene structure and function. One well publicised approach is embodied in the Human Genome Mapping Project, where the sequence of all the individual genes in the entire human genome is painstakingly being determined. At the present, however, little information can be directly retrieved on the function of the identified genes and still less about temporal and spatial expression patterns of the developing or mature organism. Other approaches, such as random cDNA sequencing, involve the sequence determination of all genes expressed in a certain tissue, or developmental stage, of an organism. Like a number of other strategies, this is time consuming and prone to numerous problems.

Although the flood of data from large scale sequencing programmes is of enormous benefit to the scientific community, one of the major problems faced by such "shotgun" approaches is the lack of specific information that can be retrieved without significantly more work on the biology of each of the individual genes.

Several other approaches have been taken by molecular biologists to obtain more specific information on the genetic background of particular biological processes. Such approaches rely on a common concept. One gene, or a subset of genes, is switched on, initiating the healthy, pathological, or developmental status of an organ or cell type.

In a large number of experimental systems the isolation of genes, on the basis of their differential expression, has been applied successfully. Differential screening and subtractive hybridisation of cDNA libraries have become well established, cf. Zimmerman *et al*. (1980) and Davis *et al*. (1979). Differential library screening works well in practice for genes that are highly expressed, but mRNAs of low abundance are difficult to isolate. Subtractive hybridisation provides a more sensitive screening, but requires large amounts of RNA. More recently RNA fingerprinting methods (often referred to as differential display or DD/RT PCR) have been added to these tools, offering attractive new features for isolating genes. RNA fingerprinting methods are PCR based and therefore do not require large amounts of RNA for experiments. In addition to this, RNA fingerprinting methods allow a large number of RNA pools to be screened for specific mRNAs simultaneously. Investigation of a wide range of pathogenic developmental stages and their controls would be possible. To date, two methods of RNA fingerprinting have proven useful for isolating genes. In 1992 Liang *et al*. published a protocol (US Patent 5,262,311), soon after a protocol from Welsh *et al*. (1992) was presented. Both methods begin with cDNA synthesis from RNA using at least one arbitrary primer for the initiation of first and second strand synthesis.

Welsh *et al*. (1992) designed a protocol in which the same arbitrary 20-mer oligo is used for first and second strand synthesis. Using arbitrary primers only a subset of the mRNAs are transcribed to cDNA. The cDNA pools are then used for a standard PCR with the same primers. One of the dNTPs in the PCR mix contains a radioactive label ($^{35}$S or $^{32}$P) for visualisation of the PCR fragments with PAGE. The Liang and Welsh methods rely on at least one small arbitrary primer for selection of specific cDNAs. As a consequence annealing temperatures are low (~40°C), and all amplified cDNA fragments originate from a certain degree of mismatch priming. Later several groups produced refinements and optimisations leading to a plethora of articles describing the usefulness of the method (Bauer and Warthoe *et al*. 1993; Warthoe *et al*. 1995; Liang and Warthoe *et al*. 1995; Rohde and Warthoe *et al*. 1996).

OBJECT OF THE INVENTION

It is an object of the present invention to provide new methods and means for investigating the expression patterns in cells, especially in eukaryotic cells. The results of such investigations may be used in drug development, gene discovery, diagnosis of diseases etc., and therefore such improved methods are highly desirable.

SUMMARY OF THE INVENTION

In its broadest scope, the invention pertains to a method for preparing a sub-divided library of amplified cDNA fragments from the coding region of mRNA contained in a sample, the method comprising the steps of

a) subjecting the mRNA derived from the sample to reverse transcription using at least one cDNA primer thereby obtaining first strand cDNA fragments,

b) synthesizing second strand cDNA complementary to the first strand cDNA fragments by use of the first strand DNA fragments as templates, thereby obtaining double stranded cDNA fragments,

c) digesting the double stranded cDNA fragments with at least one restriction endonuclease, thereby obtaining cleaved cDNA fragments,

d) ligating at least two adapter fragments to the cleaved cDNA fragments obtained in step c), so as to obtain ligated cDNA fragments, and

e) subjecting the ligated cDNA fragments obtained in step d) to a molecular amplification procedure so as to obtain amplified cDNA fragments, wherein is used, for an adapter fragment used in step d), a set of amplification primers having the general formula

$$5'\text{-Com-}N_{n1}\text{-}3' \hspace{4cm} \text{II}$$

wherein Com is a sequence complementary to at least the 5'-end of an adapter fragment which is ligated to the 3'-end of a cleaved cDNA fragment, N is A, G, T, or C, and n1 is an integer 2 0, and wherein at least one set of primers has the general formula II where n1 > 0, said at least one set being capable of priming amplification of any nucleotide sequence ligated in its 3'-end to the adapter fragment complementary in its 5'-end to Com.

The overall advantage of the invention compared to the prior art is that the resulting library of cDNA fragments contains nucleic acid sequences from all parts of cDNA which is produced in step a). Prior art techniques which i.a. rely on poly-dT cDNA priming have a tendency to only yield fragments derived from the long untranslated regions of mRNA. Furthermore, by fine-tuning of the conditions in each step, the method of the present invention results in highly specific reproduction of sequence information which is present in mRNA, even in mRNA which is only present in relatively low amounts. Furthermore, by choosing the optimum composition of endonuclease(s) it is possible to obtain cDNA fragments which are derived from a very large percentage of the total number of transcribed genes in relevant cells.

The present method allows the targeted visualisation of known genes by using primer combinations, corresponding to sequences from the gene of interest. This has the advantage that all steps of the procedure and the biological system can easily be verified. Also, very specific expression analyses can be carried out on related genes with very high homology which could not be achieved by using hybridisation technology.

Briefly, further steps in the method of the invention involve isolation of bands of interest from a gel, their cloning and sequencing. The sequence information allows re-amplification of individual bands, using primers with the appropriate 3-4 nucleotide extensions. When run on a gel, these reactions will show one, or only a few, bands per lane, giving an unequivocal determination of band identity.

Since the present technology makes use of end labelled primers for visualization, the technology can be used, both with standard technologies involving radioactivity, or with fluorescent labelled primers, without the need for further optimisation.

The invention also pertains to methods for detecting differences between expression level(s) in cells which have been subjected to different conditions, methods for diagnosing disease.

In the following is given a short discussion of terms used in the present application:

"A sub-divided library of amplified cDNA fragments" is in the present context a library of amplified cDNA fragments which is split into a number of separate pools, each pool being defined by the sequences of the termini of the amplified fragments. For example, one pool may contain amplified fragments which are all characterized by having the sequence 5'-Com-AGC- in one of the strands, whereas another pool contains amplified fragments having the sequence 5'-Com-AAT in one of the strands. For a discussion of the meaning of "Com", cf. below.

"A normalised library" is a library containing substantially equal representation of each mRNA, i.e. approximately the same number of copies of each mRNA.

"Reverse transcription" has its usual meaning in the art, *i.e.* synthesis of DNA using RNA as a template and effected by an enzyme having reverse transcriptase activity.

"Adapter fragment" is intended to mean a nucleic acid sequence containing a known sequence which can be used as template for a primer in a subsequent molecular amplification procedure such as PCR. The adapter fragment is further characterized by its ability to become integrated at the end of a cDNA fragment which has previously been cleaved with a restriction endonuclease in step c). In most cases, the restriction endonuclease leaves fragments having "sticky ends", to which the adapter fragment will anneal readily, and thereafter the adapter fragment becomes ligated to the cDNA by the action of a DNA ligase.

DETAILED DISCLOSURE OF THE INVENTION

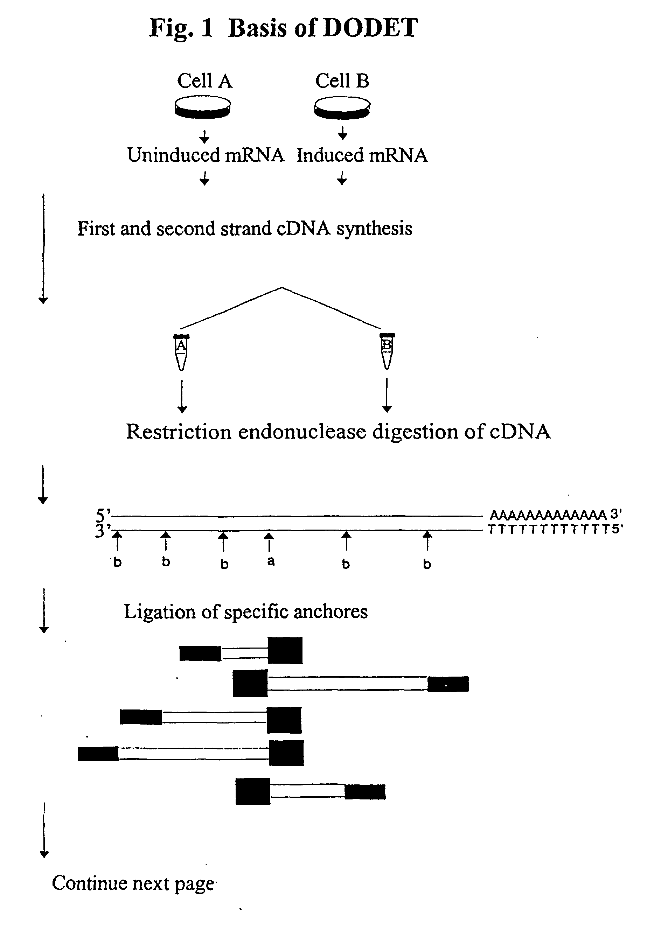

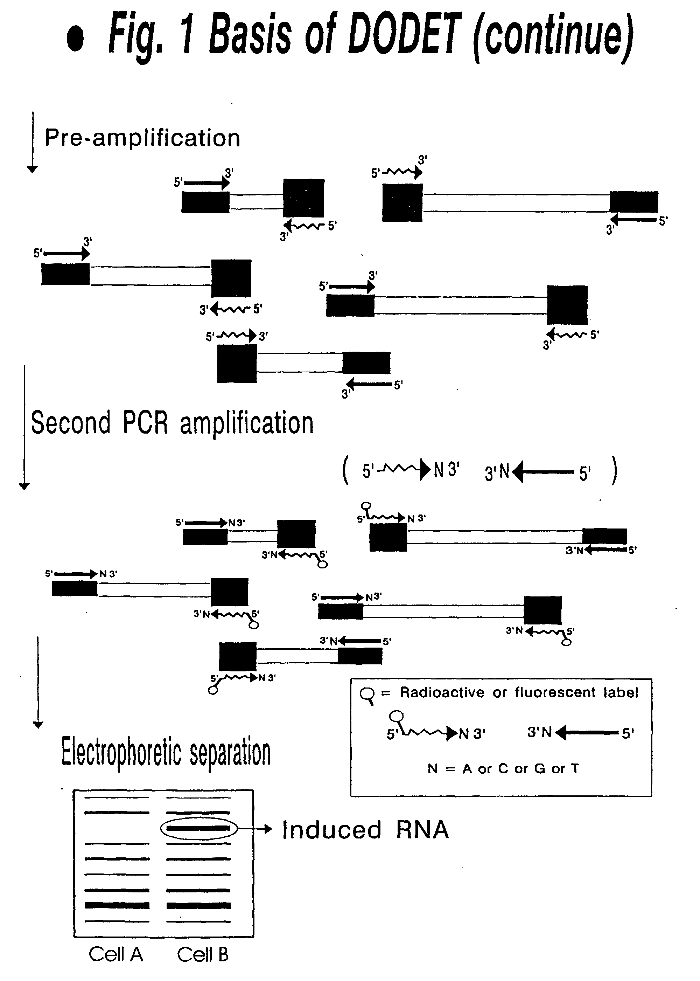

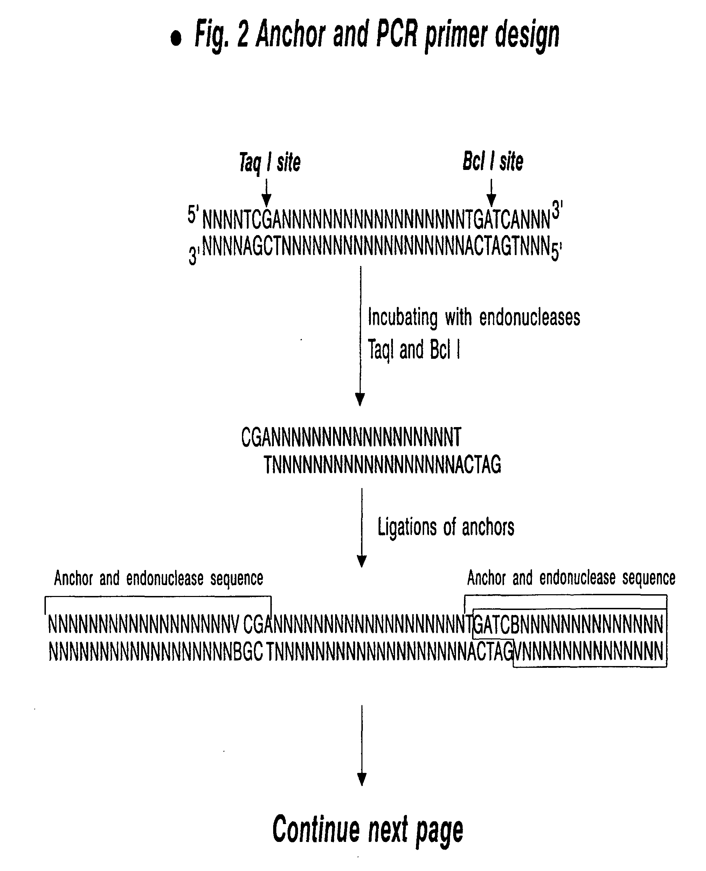

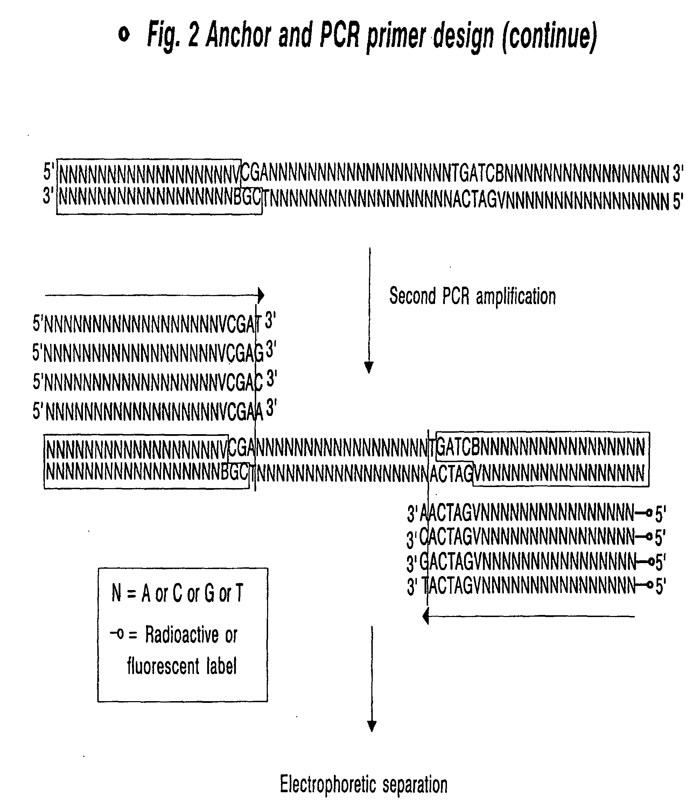

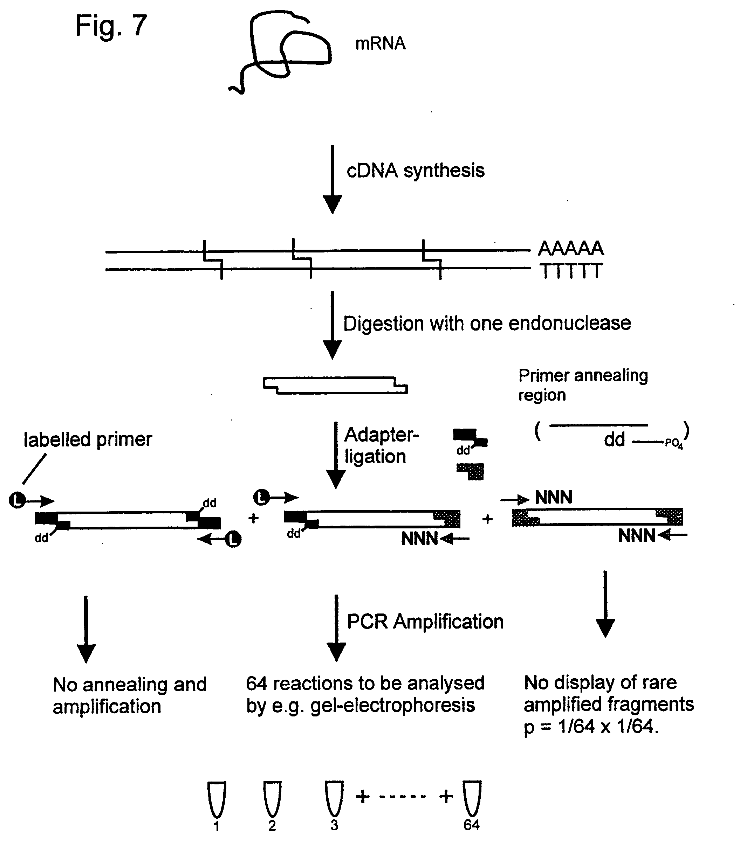

In the following, the impact of each of the steps will be discussed in detail, see Figures 1 and 7.

Step a)

The goal in step a) is to produce a mixture of first strand cDNA fragments which is optimized in its composition for carrying out the subsequent steps. A number of considerations apply:

First of all, to reduce the "background noise", it is preferred that the annealing of cDNA primer to the RNA in step a) is performed under high stringency conditions, thereby ensuring that a minimum of mismatches are introduced in the cDNA relative to the mRNA, i.e. at a temperature above 50°C.

Secondly, it is desirable to obtain copies of sequences which are derived from all parts of mRNA in order to obtain information relating to the translated part of the mRNA. Prior art methods for reverse transcription of eukaryotic material have often utilised poly-dT as cDNA primers. This strategy has, however, the disadvantage that the most efficiently reverse transcribed material is situated in the untranslated part of the genes of interest. Hence, the only parts of the mRNA which become "visible" after e.g. a PCR procedure will very often be derived from untranslated regions of the RNA. The reason for this is two effects. First of all, the poly-dT approach has the consequence that the initiation point of reverse transcription is situated very far from e.g. the start codon relating to the operon in question. Secondly, the mRNA may include structures (e.g. "hairpin" structures due to intra-chain base-pairing) which block reverse transcription and by always initiating reverse transcription at one terminus of a gene, such structures will statistically block reverse transcription of a number of translated regions.

It is in the present invention preferred to ensure that cDNAs are produced in step a) which are representatives of the entire gene, including the translated regions.

In a preferred embodiment the reaction mixture with the sample comprises a cDNA primer, said cDNA primer being sufficiently complementary to the target RNA present in the sample to hybridize therewith and initiate synthesis of a single stranded cDNA molecule complementary to said target RNA and the reaction mixture comprises an appropriate buffer which comprises all four deoxyribonucleoside triphosphates and a divalent cation selected from the group of $Mg^{+2}$ and $Mn^{2+}$ in a concentration between 0.1 0.1 and 5 mM.

The object of the method of the invention is to obtain a subdivision of the cDNA produced. When the mRNA is derived from a eukaryotic system, the at least one cDNA primer may include an oligo or poly dT tail in the 5'-end, having the general formula $5'-dT_{n2}-V_{n3}-N_{n4}-3'$, wherein dT is deoxythymidinyl, V is A, G, or C, N is A, G, C, or T, n2 is an integer $\geq 1$, n3 is 0 or 1, if n3 is 0 then n4 is 0, and if n3 is 1 then n4 is an integer $\geq 0$. It will be clear that when n3 and n4 are both zero, then the primer is an ordinary poly- or oligo-dT cDNA primer. However, when n3 is 1, then the primer is in fact a primer composition which will be able to prime the reverse transcription of any mRNA having a poly-A tail. If the original sample of RNA is subdivided, and each subpool is subjected to reverse transcription which uses one of the possible primers having the above formula where n3 is 1, then the result is a number of single stranded cDNA pools which are each different from each other in the 5'-end.

For examle, when n3 is 1, $3 \times 4^{n4}$ groups of cDNA primers are used, each group being distinct from any one of the other groups with respect to the structure $-V_{n3}-N_{n4}-$. In such an embodiment the pool of mRNA is conveniently subdivided into $3 \times 4^{n4}$ aliquots which are each subjected separately to step a) utilising one of the $3 \times 4^{n4}$ groups of cDNA primers, thereby obtaining a subdivision of the first strand cDNA into $3 \times 4^{n4}$ separate pools. Normally n4 will be 0 or 1, resulting in the provision of 3 or 12 pools, respectively.

When the starting material is not eukaryotic or when it is not the intention to necessarily set out from the part of the transcribed gene which is most remote relative to the translation start codon, the at least one cDNA primer does not include a poly or oligo dT tail in the 5'-end, or, alternatively, at least two cDNA primers are used of which at least one includes a poly or oligo dT tail in the 5'-end and of which at least one second does not include a poly or oligo dT tail in the 5'-end. Preferably, the cDNA primer which does not include a poly or oligo dT tail in the 5'end has the following structure

$$5'\text{-}N_x TTA\text{-}3' \text{ or } 5'\text{-}N_x CTA\text{-}3' \text{ or } 5'\text{-}N_x TCA\text{-}3',$$

wherein N is A, G, T, or C, and x is an integer $1 \leq x \leq 20$. It will be clear that this corresponds to cDNA priming setting out from any translation stop codon. As for the above embodiments utilising a poly- or oligo-dT tailed primer, it is, by preparing primers having all possible permutations represented in the group $N_x$, possible to compose the primers so as to correspond to any possible sequence preceding a stop codon, thereby ensuring priming of all sequences having a stop codon in their sequence.

Step b)

This step is carried out by methods well known in the art. It is, however, preferred that step b) is carried out under conditions which minimize the formation of mismatches between nucleotides in the first and second cDNA strands. The double stranded cDNA procedure can be performed according to standard methods as described in Sambrook *et al*. (1989). However since standard polymerases can have difficulty in synthesising regions containing secondary structures or with high GC-content, thermostable RNase H (Hybridase Thermostable RNase H, US 5,268,289) and thermostable rBst DNA polymerase from Bacillus stearothermophilus help overcome some of the limitations that standard polymerases (low temperature polymerases) suffer from.

Step c)

After the preparation and optional subdivision of the mRNA obtained in step b), each of the different pools of cDNA is digested with at least one restriction enzyme to produce fragments of a size which can be separated using an appropriate size fractionation method.
The choice of restriction enzyme is based largely on the frequency of the cleavage sites in a given cDNA pool. Too many cleavage sites in each cDNA fragment will result in too small fragments, and vice versa. Optimally, the at least one enzyme should cleave <u>every</u> cDNA to yield fragments of the desired size. Statistically, it is not possible to cleave every cDNA, but on the other hand a very large percentage can be cleaved by choosing a suitable enzyme or combination of enzymes. It is preferred that the method of the invention utilises at least one restriction enzyme chosen so as to ensure that at least 60% of cDNA's are cleaved, but higher percentages such as at least 65%, at least 70%, at least 75%, at least 80%, or even at least 85% are more preferred.
The invention should use restriction enzymes that leave protruding ends (sticky ends) at the termini of the DNA after digestion in step c), since this greatly facilitates the introduction of the adapter fragments in step d).
As will appear from the above, the frequency with which the restriction endonuclease cleaves is important. The at least one restriction enzyme is preferably chosen so as to cleave each complete cDNA into an average of about 3 fragments. It will be understood that some cDNAs obtained from preceding steps will not be cut at all (although this is a rare incidence when the restriction enzyme(s) is/are carefully chosen) whereas others will be cut with a high frequency. It has come out that use of a rare 4 base cutter as at the least one restriction endonuclease (such as the 4 base cutter AciI, AluI, BfaI, BstUI, Csp6I, DpnI, DpnII, HaeIII, HhaI, HinP1I, HpaII, MboI, MnlI, MseI, MspI, NlaIII, RsaI, Sau3AI, TaiI, TaqI, and Tsp509I) ensures the optimum performance of the inventive method. By use of such a rare 4 base cutter, the use of only 1 restriction enzyme in step c) is sufficient and results in superior output.
Alternatively, a combination of restriction endonucleases can be used wherein a balance of e.g. 6 base cutters and 4 base cutters ensures a reasonable distribution of fragment sizes. For instance the use of a first restriction enzyme (e.g. a 6 base cutter) which statistically cleaves at least 20% of complete cDNA derived from the mRNA sample into two subfragments, and of a second restriction enzyme (e.g. a 4 base cutter) which statistically cleaves at least 50% of said subfragments into 3 further subfragments, will also result in a series of fragments suitable for later size fractionation.

Step d)

The mixture(s) obtained in step c) are then subjected to a reaction wherein adapter fragments are added to both ends of the double stranded cDNA fragments obtained. As mentioned above, this part of the procedure is greatly facilitated by the cleaved cDNA fragments having protruding "sticky" ends, because pre-designed adapter fragments which fit to these protruding ends can easily be prepared.
The adapter (or anchor) fragments are added to the cleaved fragments in order to obtain "order in chaos" in the subsequent step. By adding known sequences to the termini of the cleaved fragments, one creates targets for specific amplification primers which can be designed specifically with the aim of amplifying sequences complying to the adapter fragments. The material thus obtained (primary template) can be pre-amplified, using primers complementary to the ligated adaptor sequences, giving rise to secondary template. The pre-amplification of primary template allows virtually unlimited amounts of template to be produced from one RNA preparation, avoiding the need for repeated isolations.

The adaptor sequence is thus selected so as to serve as the starting point for DNA polymerisation in e.g. a PCR reaction. The adaptor sequences are constructed in such a way that the specific endonuclease sites are not regenerated after ligation of said adaptor.

At least one termination fragment is also ligated to the 3'-end of single strands of cleaved cDNA fragments, said at least one termination fragment introducing a block against DNA polymerization in the 5'→3' direction setting out from the at least one termination fragment and said at least one termination fragment being unable to anneal to any primer of the at least two primer sets in step f) during the molecular amplification procedure.

The above is a very important procedure when combined with the use of detection effected by labelled primers in the amplification step, wherein only one member of the pair of primers is labelled whereas the other is designed to split up the amplified products according to their base composition adjacent to the adapter fragment. One important feature is that a single stranded cDNA fragment which has been provided with a termination fragment will not be amplified, because no primers will be able to anneal to the products of a first round polymerisation wherein such a fragment was the template, see Figure 7. Secondly, the approach opens for the possibility of removing background "noise" in a subsequent detection phase.

Normally, the at least one termination fragment comprises or is a chemically modified nucleotide sequence, such as for instance a nucleotide sequence which comprises a dideoxynucleotide in the 3'-end; this termination technique is well-known from e.g. the chain-termination sequencing technique according to Sanger. Under normal circumstances, the dideoxynucleotide should, according to the invention, be covalently attached to the nucleotide strand so as to avoid loss of the dideoxynucleotide during subsequent rounds of amplification. Superior stabilisation is attained if the dideoxynucleotide is phosphorylated.

As mentioned above, the ligation of adapter and/or termination fragments to the cleaved cDNA fragments in step d) is conveniently achieved by annealing the adapter fragments to sticky ends of the cDNA resulting from the cleavage in step c) and subjecting the product to the action of an enzyme having DNA ligase activity. Any suitable DNA ligase known in the art can be used.

Step e and f)

Step e) of the method of the invention results in the final sorting of the modified cDNA fragments from step d).

The primers having the structure of formula I (step f) are designed so as to selectively amplify synthesized double stranded cDNA fragments obtained in step b) or predefined subsets of the adapted fragments obtained in step d). A number of ways this can be done may be envisaged, but the main strategy is to prime amplification in a series of separate reactions where the nucleotide sequence of one primer in one reaction ensures that the amplified products of that reaction are different from those obtained from any of the other reactions and that all the reactions result in amplification of all fragments obtained from step d), respectively.

Even though the at least one set of amplification primers of formula I wherein has a n1 which is $\geq 0$, it is preferred that n1=1, n1=2, n1=3, or n1=4 in one of the primers, because the number of primer fragments to be used in the reactions in order to cover all possible nucleotide stretches adjacent to the Con or adapter fragment is easily manageable. For instance, if n1=5, it would be necessary to use $4^5$=1024 different primers in order to obtain amplification of all possible nucleotide sequences adjacent to the relevant adapter fragment, and since the preferred embodiment of the invention requires that each such primer is used in a separate reaction, the work involved would be problematic.

It is also preferred that in one of the primers n=0, and it is especially preferred that this primer is labelled, in order to facilitate determination of the amplified fragments.

Hence, in the most preferred embodiments of the invention, the adapted cDNA fragments are amplified in a number of separate reactions wherein a labelled primer is used (which is normally identical in all reactions) and at least one non-labelled primer which is a member of the set of primers described above where n>1. It is preferred that this set of amplification primers of formula I wherein n1>0 comprises all possible combinations and permutations of A, G, T, and C in the group $N_{n1}$, since this will ensure that all possible cDNA fragments can be amplified by the set.

Hence, the ligated cDNA fragments are sub-divided into a number of pools prior to the molecular amplification in step f), and each pool is subjected to the amplification using a subset of the set of amplification primers, and in the most preferred embodiment the ligated cDNA fragments of step d) are subdivided into $4^{n1}$ pools which are each subjected separately to step f) wherein is used one amplification labelled primer as described above (n1=0) and one primer from the set of amplification primers as defined above (n>0), said one primer being distinct from any one of the primers used for amplifying any of the other pools. By using this approach, the originally reverse transcribed and cleaved cDNA fragments are subdivided into $4^{n1}$ pools which can each be subjected to further steps.

## Further steps and applications

The material obtained from the above-described series of reactions can now be utilised in a number of ways. Normally, a further step of separating amplified fragments obtained from the molecular amplification procedure is performed. This yields a mixture of amplified fragments which are separated e.g. by size separation, by mobility in a gel electrophoresis or by any suitable chromatographic method. Furthermore, a step of identification (e.g. by visualization of these separated fragments) is normally carried out for "book-keeping purposes"; the separated mixture of fragments will normally be compared to some kind of reference which may be material derived from the same or another cell type.

Visualization of the separated fragments can, as mentioned above, be achieved by one of the primers in the amplification reaction being labelled, but other methods are of course available. For instance, a specifically labelled probe which e.g. binds to one of the adapter sequences will visualise the fragments, but also labelled nucleotides which have been incorporated in the fragments during the amplification procedure (e.g. a PCR) will of course be a suitable means for detection (e.g. by incorporating radioactive or fluorescent alpha dNTP into the cDNA fragment during PCR, where N = A, C, T, U or G).

However, it is preferred that visualisation of specific RNA Derived Fragments (RDFs) is achieved using primers which are radioactively or fluorescently labelled and are homologous to the adaptors. The comparatively high annealing temperatures (touch-down from 65°C to 56°C) which are preferably used ensure that polymerisation events will predominantly originate from perfect priming of adapter sequences and adjacent selective bases. Band intensities are largely a function of initial template concentration, whereas band intensities of the original Differential Display methods are dependent on the quality of the match between the individual template and primer. The visualisation of rare mRNAs using the present inventive methods will be less hampered by the over-representation of signal from highly abundant mRNAs. As in the case of arbitrary priming, the mismatch amplification and abundant RDFs always out-compete the amplification of rare fragments base pair perfectly. Our experiments suggest that as few as 100 molecules can be routinely detected in a given template. This corresponds to less than 1 transcript per cell in the original tissue.

Another part of the invention pertains to the use of the inventive method for comparing expression levels in different cells. One way of doing this is to determine the change in expression, compared to the expression in a reference cell or reference group of cells, of an expression product in a cell or group of cells which has been subjected to a first set of conditions influencing the expression pattern of said cell or group of cells, said reference cell or group of cells being subjected to a second set of conditions, the method comprising providing an RNA-containing sample from the cell or group of cells and subjecting the sample to the method of the invention for sub-division, thereby obtaining data describing the amplified cDNA fragments derived from the sample, providing reference data describing amplified cDNA fragments derived from an RNA-containing reference sample from the reference cell or reference group of cells, the reference data being obtained by having previously subjected the reference sample to the method of the invention, subsequently performing a comparison of the data and the reference data to identify the cDNA fragments which are expressed at different levels in the two data sets, and thereafter using the differentially expressed cDNA fragments to determine which expression products are subject to a change in expression level. In other words, the method of the invention is carried out twice on the basis of two different RNA samples derived from cells subjected to differing conditions.

Normally, the data and reference data are selected from the group consisting of the apparent molecular weights of the amplified DNA fragments, the $M_r$ of the amplified DNA fragments, the absolute amount of the amplified DNA fragments, and the relative amounts of the amplified DNA fragments. The reference data can further be extracted from a database containing the reference data defined above and optionally further information relating to the genetic origin of each amplified cDNA fragment from the reference.

Related to the above, the invention also allows for diagnosis of disease which is characterized by a deviating (increased or reduced) expression level of at least one expression product in at least one cell type, the method comprising providing an RNA-containing sample derived from the at least one cell type, subjecting the sample to the method of the invention thereby obtaining data describing the amplified cDNA fragments derived from the sample, providing reference data describing amplified cDNA fragments derived from a RNA-containing reference sample derived from the same type of cell from a subject not suffering from the disease, the reference data being obtained by having previously subjected the reference sample to the method according to the invention, and subsequently performing a comparison of the data and the reference data with respect to those cDNA fragments which are known to be related to the disease, and assessing whether a significant difference in the data and reference data exists so as to establish whether the expression level of the expression product deviates or not.

As for the embodiment above, also here the data and reference data are selected from the group consisting of the apparent molecular weights of the amplified DNA fragments, the $M_r$ of the amplified DNA fragments, the absolute amount of the amplified DNA fragments, and the relative amounts of the amplified DNA fragments, and also here the reference data can be extracted from a database containing the reference data defined above and optionally further information relating to the genetic origin of each amplified cDNA fragment from the reference.

The mixtures of amplified fragments obtained from step f) of the method of the invention may also be used for preparing a surface (chip) coated with cDNA fragments. This can be done by

- subjecting an RNA-containing sample to the subdivision method of the invention including separation steps, and
- transferring the separated amplified cDNA fragments to a chip surface adapted to stably bind the separated amplified cDNA fragments while maintaining the spatial relative distribution pattern thereof.

Alternatively, such a chip can be prepared by

- subjecting an RNA-containing sample to the method of the invention without performing the separation, and thereafter
  separating, by electrophoresis, the thus obtained amplified cDNA fragments on a particular surface adapted to stably bind the separated amplified cDNA fragments while maintaining the relative distribution pattern after electrophoresis. In this embodiment, the electrophoresis is preferably in the form of microelectrophoresis.

Transfer to the surface is preferably accomplished by a electrophoretic blotting technique, and/or by well-known photo-activated organic or inorganic chemistry coupling techniques.

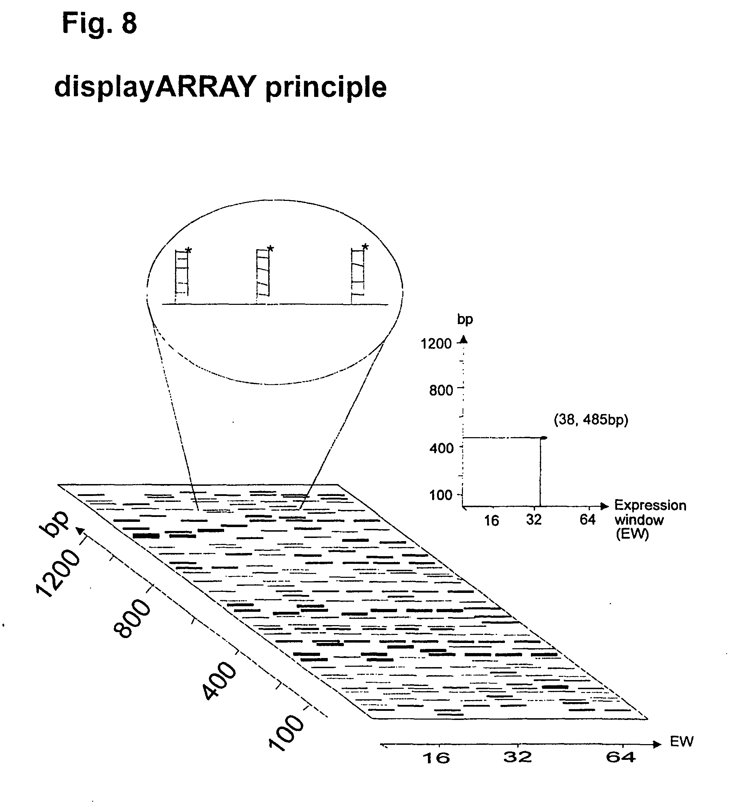

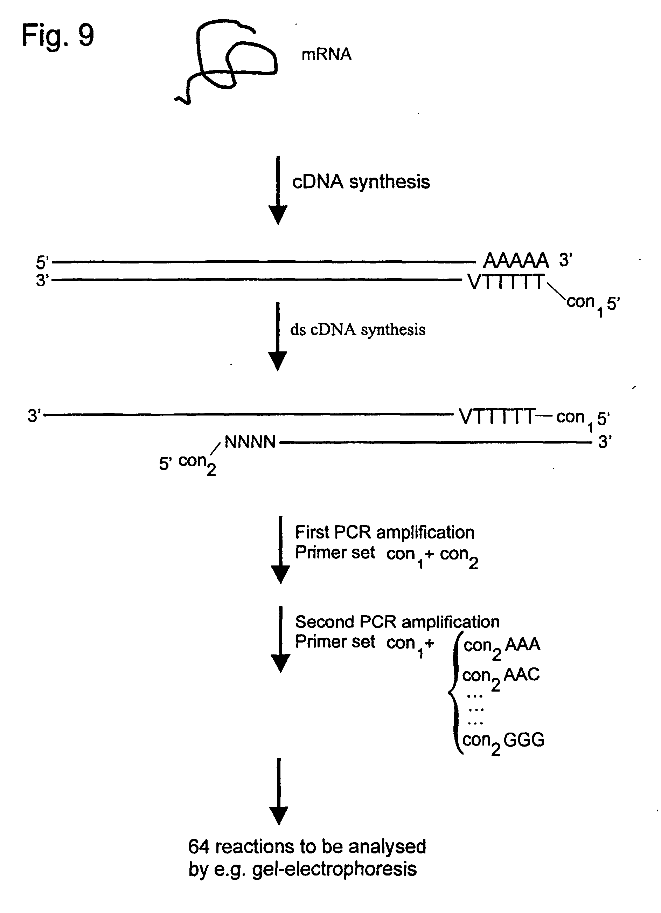

The invention also pertains to a surface obtainable by the above-mentioned method for the preparation thereof. Such surfaces are considered novel and inventive, since known "DNA chips" rely on specific introduction of an array of nucleic acid fragments of known structure, whereas the present method provides for "a semi-array" containing cDNA fragments characterizing a specific "situation" for a specific cell type, according to Figure 8.

Such a surface can i.a. be used for screening for genes within a gene family. The "array chip" is provided and thereafter a labelled probe (which is a representative of a gene family) is allowed to hybridize to the chip under low stringency i.e. under conditions as described at pages 94-106 in "Nucleic acid hybridisation. A practical approach" edited by BD Hames & S J Higgins, IRL Press. A number of fragments coupled to the chip will hybridize to the probe, and these fragments can subsequently be identified, isolated and sequenced/characterized in order to determine whether they are representatives of the same gene family.

Another use of such "semi-arrays" is for determining the difference in expression pattern between a first cell or type of cells and a second cell or type of cells, the method comprising providing samples of labelled RNA or cDNA from the first and second cells or cell types and subsequently contacting each of these samples with a chip surface as described above, and subsequently detecting the amount and distribution of bound labelled RNA or cDNA from each sample.

Under all circumstances, the chip surface with the cDNA bound thereto can e.g. be produced by the methods described in EP-0 654 061.

Yet another part of the invention pertains to a method for screening for interactions between a pre-selected protein and a polypeptide fragment, the method comprising preparing a sub-divided library of amplified cDNA fragments resulting from step e), optionally adapting the terminals of the members of the library so as to facilitate insertion in a vector, inserting the fragments into vectors, transforming a population of suitable host cells with the vectors, culturing the host cells under conditions which enable expression of correctly inserted cDNA fragments by the host cell, and subsequently assaying polypeptide fragments encoded by the inserted cDNA fragments for interaction with the pre-selected protein.

One convenient way of achieving this is by way of a two-hybrid technique, wherein the host cells are eukaryotic cells (such as fungal cells, especially yeast cells) which are mated or transfected with nucleic acid material encoding the pre-selected protein, successful mating/transfection of the cell(s) resulting in a cell or cells wherein the interaction between the pre-selected protein and a polypeptide fragment gives rise to a detectable signal.

Such methods have recently attracted a great deal of attention, *i.a.* as a consequence of the disclosure in Fromont-Racine *et. al.*, Nature Genetics **16**, 277-282 (1997), which is incorporated by reference herein.

One convenient system for providing the detectable signal is by use of Green Fluorescent Protein, disclosed in EP-A-0 569 170, wherein changes in fluorescent spectrum due to interactions are used as reporter.

DESCRIPTION OF THE PREFERRED EMBODIMENTS

First, the drawing will be briefly described.
Fig. 1

Basis of Display Of Differentially Expressed Transcripts.

Fig. 2

Anchor and PCR primer design.

Fig. 3

An autoradiogram of a DODET gel using the cellular set-up described in Example 1; rat pheochromocytoma PC12 cells were stimulated with the Nerve Growth Factor (NGF) and Epidermal growth factor (EGF).

Lanes 1 - 24, reverse transcription using the anchored poly T primer 5'-$T_{25}$AA-3'

Lanes 25 - 48, reverse transcription using the anchored poly T primer 5'-$T_{25}$GC-3'

Lanes 1, 5, 9, 13, 17, 21, 25, 29, 33, 37, 41, 45 represent the PC12 cells not treated.

Lanes 2, 6, 10, 14, 18, 22, 26, 30, 34, 38, 42 and 46 represent the PC12 cells treated with the NGF factor for 60 minutes.

Lanes 3, 7, 11, 15, 19, 23, 27, 31, 35, 39, 43 and 47 represent the PC12 cells treated with the NGF factor for 90 minutes

Lanes 4, 8, 12, 16, 20, 24, 28, 32, 36, 40, 44 and 48 represent the PC12 cells treated with the EGF factor for 90 minutes.

Lanes 1 - 48, using the following pairs for the pre-PCR amplifications:

*Taq*I pre-amplification primer: 5'-CAGCATGAGTCCTGACCGA

*Bcl*I pre-amplification primer: 5'-CTCGTAGACTGCGTACCGATCA

For the second PCR amplification the following primer pairs were used:

Lanes 1-4

```
5'-CATGAGTCCTGACCGAA
5'-GACTGCGTACCGATCAA        (5' end labelling)
```

Lanes 5 - 8

```
5'-CATGAGTCCTGACCGAA
5'-GACTGCGTACCGATCAC        (5' end labelling)
```

Lanes 9 - 12

```
5'-CATGAGTCCTGACCGAA
5'-GACTGCGTACCGATCAG        (5' end labelling)
```

Lanes 13 - 16

```
5'-CATGAGTCCTGACCGAA
5'-GACTGCGTACCGATCAT        (5' end labelling)
```

Lanes 17 - 20

```
5'-CATGAGTCCTGACCGAC
5'-GACTGCGTACCGATCAA          (5' end labelling)
```

Lanes 21 - 24

```
5'-CATGAGTCCTGACCGAC
5'-GACTGCGTACCGATCAC          (5' end labelling)
```

Lanes 25 - 48

Repeated primer combination from lanes 1 - 24

Fig. 4a
Northern Blot of RDF01 sequence from cellular total RNA. a) PC12 cells not treated, b) NGF treatment for 60 minutes, c) NGF treatment for 90 minutes, and d) EGF treatment for 90 minutes.

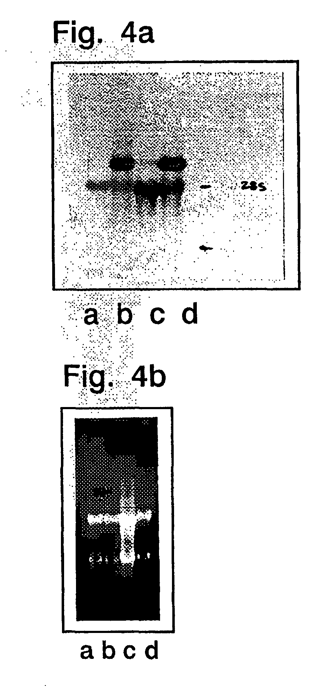

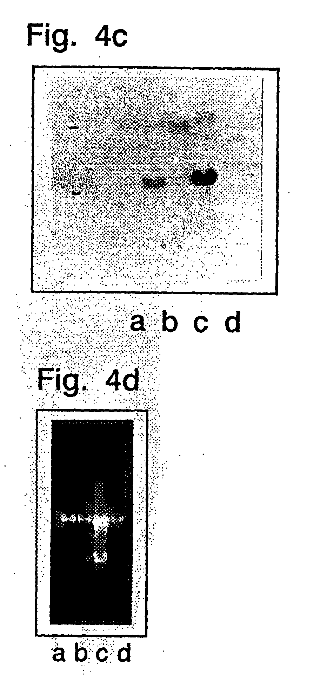

Fig. 4b
Loading control, RNA extracts were electrophoresed on a 1.2% agarose gel containing ethidium bromide, used as a control to determine the relative concentration of RNA in each lane, a, b, c, d same as in Figure 4a

Fig. 4c
Northern Blot of RDF02 sequence from cellular total RNA. a) PC12 cells not treated, b) NGF treatment for 60 minutes, c) NGF treatment for 90 minutes, and d) EGF treatment for 90 minutes.

Fig. 4d
Loading control, RNA extracts were electrophoresed on a 1.2% agarose gel containing ethidium bromide, used as a control to determine the relative amount of RNA in each lane, a, b, c, d same as in Figure 4c

Fig. 5
Searching for genes modulated by a growth factor.

Lane 1          Size marker in bp (150 bp, 200 bp, 250 bp).
Lanes 2-6       Amplification primer 5'-Com-$N_{n1}$-3' where $N_{n1}$ is GAA
Lanes 7-11      Amplification primer 5'-Com-$N_{n1}$-3' where $N_{n1}$ is GAC
Lanes 12-16     Amplification primer 5'-Com-$N_{n1}$-3' where $N_{n1}$ is GAG
Lanes 17-21     Amplification primer 5'-Com-$N_{n1}$-3' where $N_{n1}$ is GAT
Lanes 22-26     Amplification primer 5'-Com-$N_{n1}$-3' where $N_{n1}$ is GCA

In lane 11 a downregulation is observed after 6 days treatment, whereas in lane 16 an upregulation is observed after 6 days treatment. Both modulations are due to the growth factor, since regulation is seen only when the active growth factor is present.
Fig 6
Searching for genes involved in bacterial resistance.

Lane 1:         Size marker in bp (150 bp, 200 bp, 250 bp, 300 bp).
Lanes 2-5       Amplification primer 5'-Com-$N_{n1}$-3' where $N_{n1}$ is GAA
Lanes 6-9       Amplification primer 5'-Com-$N_{n1}$-3' where $N_{n1}$ is GAC
Lanes 10-13     Amplification primer 5'-Com-$N_{n1}$-3' where $N_{n1}$ is GAG
Lanes 14-17     Amplification primer 5'-Com-$N_{n1}$-3' where $N_{n1}$ is GAT
Lanes 18-21     Amplification primer 5'-Com-$N_{n1}$-3' where $N_{n1}$ is GCA
Lanes 22-25     Amplification primer 5'-Com-$N_{n1}$-3' where $N_{n1}$ is GCC

In lanes 8-9 a downregulation is observed, in lanes 20-21 an upregulation is observed. Both gene modulations are potential genes involved in the resistance to the Bacteriamycin, Inosin.

Fig. 7

Principle of the technology used in Examples 4 and 5.

After ds-cDNA synthesis the DNA is digested with one 4 base pair endonuclease and anchors are ligated to the ds-cDNA ends. Using special design primers the expression profiles are obtained by amplifying the mRNAs in different expression windows (sub-fractions). The number of expression windows depends on the complexity of the sample i.e. 64 expression windows in eukaryotic.

Fig. 8

Principles of a gene discovery DNA surface (a DNA chip).

After size separation of the DNA fragments, the DNA fragments are transferred to a nylon membrane using an electrophoretic principle. The membrane is hybridized with a complex DNA probe generated using the principle of the invention. Alternatively the membrane can be hybridized with one single gene to identify new members of a particular gene family. The membrane are in the x coordinates separated in 64 expression windows, and in the y coordinates separated in base pair size (from 50 base pair to 1200 base pair) according to principle described in figure 7.

Fig. 9

Principle of generation 64 pools of 3' END cDNAs

Step 1

Production of single stranded cDNA using $5'-con_1-T_nV$ oligonucleotide where $con_1$ is an oligonucleotide between 1-100 nucleotide, n is between 5-40 and V is a mixture of A, C and G.

Step 2

Double stranded cDNA synthesis are produced using $5'Con_2N_x$ where $con_2$ is an oligonucleotide between 1-100 nucleotide, x is between 1-10, and N is a mixture of A, C, T and G. The ds-cDNA synthesis is synthesized by Klenow enzyme with the above-described oligonucleotide.

Step 3

Pre-amplification of double stranded cDNA to amplify the double stranded cDNA, the cDNA is PCR amplified using a combination of $con_1$ and $con_2$ primers.

Step 4

The pre-amplified cDNA is further amplified and separated in 64 pools using a combination of a labeled $con_1$ and 64 $con_2$NNN primers in a PCR amplification procedure, where NNN are combined in 64 different ways using the nucleotides A, T, G and C.

Step 5

Each of the 64 pools is separated using the Page electrophoresis principle.

EXAMPLES

In order to verify the functionality of the invention, examples are described below in which a developmental eukaryotic cellular system, pheochromocytoma PC12, was employed.

Nerve Growth Factor (NGF) induces growth arrest and neurone outgrowth in the *in vitro* PC12 cell system. Other growth factors, such as epidermal growth factor (EGF), support survival and stimulate growth. NGF-induced genes, include the immediate early genes, which encode transcription factors, such as c-fos and c-myc. The products of the immediate early genes are thought to be involved in regulating the expression of genes, associated with the neuronal phenotype for example neurofilaments, peripherin, GAP43 and transin.

In order to identify new early genes involved in neuronal differentiation and proliferation, the following DODET method is used for identify such genes.

In the following examples, it is demonstrated how efficiently the method of the invention can be applied to such cellular systems.

EXAMPLE 1

The rat pheochromocytoma PC12 cells were grown (*in vitro*) in the presence and absence of Nerve Growth Factor (NGF) and epidermal growth factor (EGF) under growth conditions described elsewhere (Saltiel *et al*. 1996).

The total RNA was isolated using the standard single-step method by Chomczynski and Sacchi according to Sambrook *et al* 1989.

Total RNA concentration was determined spectrophotometrically and then adjusted to 0.2 µg/µl. This RNA was used directly in the Northern analysis.

For DODET 4 x 0.5 µg total RNA was reverse transcribed in separated pools using the primer 5'-$T_{25}$AA-3'. The same procedure was performed using the 5'-$T_{25}$GC-3' poly-dT anchored primers, giving a total of 2 x 4 x 0.5 µg of RNA.

First strand synthesis

| | |
|---|---|
| 20.0 µl | total RNA amount between 0.3 to 1.0 µg RNA |
| 3.0 µl | 5'-$T_{25}$AA-3' Conc. 100 ng/µl or 5'-$T_{25}$GC-3' Conc. 100 ng/µl |
| 5.0 µl | 10 x cDNA buffer (buffer B from Epicentre Technologies # R19250) |
| 2.0 µl | dNTPs (25 mM from Pharmacia Biotech) |
| 1.0 µl | SuperScript II RT (200 U/µl) (Gibco BRL # 18064-014) |
| 5.0 µl | Retrotherm RT (1 U/µl) (Epicentre Technologies #R19250) |
| 14.0 µl | $H_2O$ |

To obtain high specificity, the cDNA reaction was incubated at 50°C for 30 minutes followed by 1 hour incubation at 70°C.

Second strand synthesis:

To the first strand reaction, add the following components

| | |
|---|---|
| 15.0 µl | 10 x cDNA buffer |
| 3.0 µl | Hybridase Thermostable RNase (1 U/µl) (Epicentre Technologies #H39050) |
| 1.0 µl | rBst thermostable DNA polymerase (1 U/µl)) (Epi-centre Technologies #BH1100) |
| 81.0 µl | $H_2O$ |

Incubate at 65°C for 1 hour.

The resulting double stranded cDNA was phenol extracted and precipitated and resuspended in 20 µl of $H_2O$. Half of this volume was checked on gel; if a smear between 100 bp and 3000 bp was observed, the rest of the cDNA was used for DODET template production. The resulting cDNAs were digested with 10 U of each of the thermostable restriction enzymes *Taq*I and *Bcl*I at 50°C for 2 hours. To this mixture, DODET adapters were added and ligated to the ends of the restriction fragments with $T_4$ DNA ligase (1U) resulting in the primary template. 8-15 cycles of non-radioactive pre-amplification, using primers complementary to the DODET adapters, were performed on a small aliquot (1/10th volume) of the primary template (94°C denaturation; 30 s, 56°C annealing; 30 s, 72°C polymerisation; 1 min). The products of the amplification (termed secondary template) were also checked on a 1.5% agarose gel. As expected, fragment sizes were predominantly between 100 bp and 1000 bp. All amplification reactions were carried out on a PE-9600 thermocycler using Taq DNA-polymerase, both from Perkin Elmer Corp. (Norwalk, CT, USA). The final template was then diluted 10 fold with $H_2O$.

The adapters ligated to the restriction fragments, the pre-amplification and active PCR are given below:

*Taq*I adapter:    5'-CAGCATGAGTCCTGAC

                   TACTCAGGACTGGC-5'

*TaqI* pre-amplification primer: 5'-CAGCATGAGTCCTGACCGA

*TaqI* amplification primer:      5'-CATGAGTCCTGACCGAN
(N = A or C or G or T)

*BcI*I adapter:   5'-CTCGTAGACTGCGTACC
CTGACGCATGGCTAG-5'

*Bcl*I pre-amplification primer: 5'-CTCGTAGACTGCGTACCGATCA

*Bcl*I amplification primer:  5'-GACTGCGTACCGATCAN
(N = A or C or G or T)

For PCR all the different combinations of one extension (denoted as N above) were available, giving a total of $4^2$ primer combinations. All oligonucleotides were obtained from DNA Technology (Aarhus, Denmark).

Radioactive labelling of the *Bcl*I primer was performed using 1U of $T_4$ polynucleotide kinase. Thermocycling was carried out essentially as described above but with 35 cycles and including an 11 cycle touch-down (the annealing temperature was reduced from 65°C to 56°C in 0.7°C steps for 11 cycles and subsequently maintained at 56°C for 23 cycles). Samples were then boiled after the addition of dye and 50% formamide and separated on a 5% polyacrylamide sequencing type gel (GIBCO BRL Life Technologies Inc., Gaithersburg, MD, USA). All gels were run at standard conditions, such that the 70 bp marker was 3 cm from the bottom of the gel, giving good resolution between 70-800 bp. Gels were then dried directly onto Whatman 3M paper on a slab gel dryer. Labelled DNA fragments were visualised by autoradiography. Gels and films were positionally marked prior to development. The 1 base selective extensions were chosen empirically to yield approximately 50 radioactively labelled fragments per lane.

Bands, identified on the autoradiogram as interesting, were lined up with markings on the film and the dehydrated gel and were excised. Excised fragments were monitored for activity. The gel fragments were isolated using GENECLEAN (BI0101, California USA). DNA was then recovered according to the manufacturer's recommendations. DNA fragments could then be reamplified using the same PCR conditions and primers as used in the initial PCR; however, 15 cycles generally yielded sufficient product for cloning. Cloning was achieved using unpurified PCR product and the vector display-p123T (Display Systems Biotech, USA). Conditions were used as recommended by the manufacturer.

EXAMPLE 2

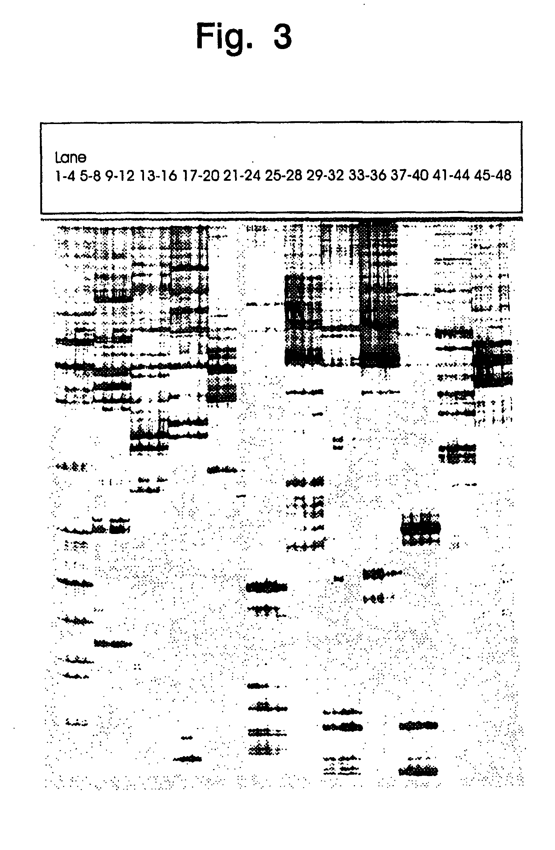

Figure 3 shows a typical DODET gel produced by amplification of template derived from treatment of PC12 cells with NGF or EGF.

Total RNA was reverse transcribed with the 5'-$T_{25}$AA-3' and $T_{25}$GC-3' poly-dT anchored primers, and after anchor ligation pre-amplification with *Bcl*I and *Taq*I pre-amplification primer pairs was performed.

6 out of 16 possible primer combinations are shown, using 1 selective base, at each restriction enzyme site (Figure 3). The largest visible products (Figure 3) are approximately 1000 bp in size and the lower end of the gel corresponds to approximate 100 bp. In this size window an average of 50 bands can be scored for each primer combination. In Figure 3, various expression patterns can be detected.

Due primarily to the stringent conditions possible in DODET, resolution of the banding pattern is high while the level of background remains at acceptable levels (Figure 3). Furthermore, quite radical changes in the intensity of individual bands over the treatment period do not seem to affect the patterns of other bands in the same lane.

It is, therefore, possible to conclude that the PCR remains proportionally independent on the concentration of individual substrates in the reaction.

The use of an optimised combination of standard protocols described above for isolating, re-amplifying and cloning individual RDFs, has allowed the identification of a number of transcripts associated with differentiation and proliferation events.

Four RDFs were isolated for further analysis, Figure 3, bands a, b, c and d.

Sequence analysis revealed that RDF a = RDF b and RDF c = RDF d, as illustrated in Figure 3.

In all cases appropriate terminal sequences with the correct 1 selective base extensions used in the PCR could be retrieved, demonstrating the stringency and fidelity of the system (data not shown).

EXAMPLE 3

During scanning of the PC12 cellular systems treated with NGF or EGF with different primer combinations, two RDFs (designated RDF01 and RDF02) exhibiting a differential expression during the NGF treatment were isolated (RDF01 = RDF a = RDF b and RDF02 = RDFc = RDF d. in Figure 3).

After re-amplification, sub-cloning and DNA sequencing, further DNA analysis revealed two unknown RDFs upregulated after 60 minutes NGF treatment or 90 minutes EGF treatment in the PC12 cellular system. The nucleotide sequences of both RDF01 and RDF02 show less than 10% homology to any existing gene in the GeneBank or EMBL databases.

The expression of RDF01 and RDF02 was further analyzed using Northern blot (Figure 4a - 4d). Here, transcripts could clearly be detected at 60 minutes NGF treatment or 90 minutes EGF treatment of the PC12 cells, confirming the results obtained using the DODET method, as illustrated in Figure 3.

Experiments to clone the full length of RDF01 and RDF02, and biological characterisation of their involvement in the differentiation and proliferation, of the PC12 cellular system are currently under investigation.

EXAMPLE 4

*Searching for genes modulated by a growth factor*.

A human cell line was treated with a growth factor and RNA was isolated a various time points as indicated below.

| 1 | Cell without any treatment (lanes 2, 7, 12, 17, 22) |
| 2 | Cell treated with helper agent, 1 day (lanes 3, 8, 13, 18, 23) |
| 3 | Cell treated with helper agent and growth factor, 1 day (lanes 4, 9, 14, 19, 24) |
| 4 | Cell treated with helper agent, 6 days (lanes 5, 10, 15, 20, 25) |
| 5 | Cell treated with helper agent and growth factor, 6 days (lanes 6, 11, 16, 21, 26) |

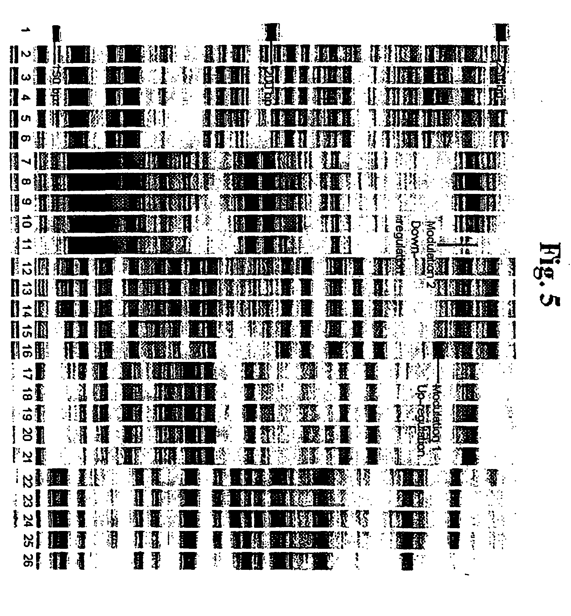

Human RNA was isolated and the gene discovery analysis was performed essentially as described in the legend to Fig. 3. 5 out of 64 amplification primers are shown in Fig. 5, each covering a certain portion of the mRNA pool in the human cell line. The expression analysis was performed on an ALFexpress, an automated fragment analyzer from Pharmacia Biotech, using a Cy5 label.

In lane 11 a downregulation is observed after 6 days of treatment. In lane 16 an upregulation is observed after 6 days of treatment. Both modulations are due to the growth factor, since regulation is only observed with the active growth factor present.

EXAMPLE 5

*Searching for genes involved in bacterial resistance to antibiotics*.

A *Listeria monocylogia* strain was treated with the Bacteria-mycin, Inosin. RNA from a strain resistant to Inosin was further investigated.

| 1. | Bacterial clone 1 without any treatment (lanes 2, 6, 10, 14, 18) |
| 2. | Bacterial clone 2 without any treatment (lanes 3, 7, 11, 15, 19) |
| 3. | Bacterial clone 3 resistant to Inosin (lanes 4, 8, 12, 16, 20) |
| 4. | Bacterial clone 4 resistant to Inosin (lanes 5, 9, 13, 17, 21) |

Bacterial RNA was isolated by standard techniques and the gene discovery analysis was performed according to Example 4 and Fig. 5, with the exception that a 5'-NNNNNNYYA primer was used for first strand synthesis.

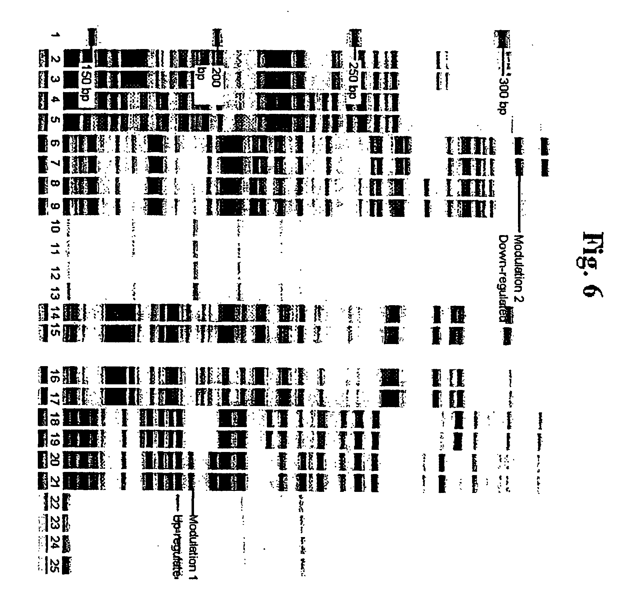

6 of 64 amplification primers are shown in Figure 6, each covering a certain portion of the mRNA pool in the prokaryotic cell system. The expression analysis was performed on an ALFexpress, an automated fragment analyzer from Pharmacia Biotech, using a Cy5 label.

In lanes 8 and 9 a downregulation is observed and in lanes 20 and 21 an upregulation is observed. Both gene modulations are potential genes involved in the resistance to Inosin.

**Claims**

1. A method for preparing a normalized sub-divided library of amplified cDNA fragments from the coding region of mRNA contained in a sample, the method comprising the steps of

    a) subjecting the mRNA derived from the sample to reverse transcription using at least one cDNA primer, thereby obtaining first strand cDNA fragments,

    b) synthesizing second strand cDNA complementary to the first strand cDNA fragments by use of the first strand DNA fragments as templates, thereby obtaining double stranded cDNA fragments,

    c) digesting the double stranded cDNA fragments with one restriction endonuclease, said restriction endonuclease leaving protruding ends (sticky ends) of similar size at the termini of the DNA after digestion, thereby obtaining cleaved cDNA fragments,

    d) adding at least two adapter fragments containing known sequences to the cleaved cDNA fragments obtained in step c), said at least two adapter fragments being able to bind specifically to the sticky ends of the double stranded cDNA pro-duced in step c), the one adapter fragment being able to anneal to the primer having formula I in step e), the second adapter fragment being a termination fragment introducing a block against DNA polymerization in the 5'→3' direction setting out from said termination fragment and said termination fragment being unable to anneal to any primer of the at least two primer sets in step e) during the molecular amplification procedure, the at least two adapter fragments being ligated to the cleaved cDNA fragments obtained in step c), so as to obtain ligated cDNA fragments,

    e) sub-dividing the ligated cDNA fragments obtained in step d) into $4^{n1}$ pools where $1 \leq n1 \leq 4$, and

    f) subjecting each pool of ligated cDNA fragments obtained in step e) to a molecular amplification procedure so as to obtain amplified cDNA fragments, wherein is used, for an adapter fragment used in step d), a set of amplification primers having the general formula

    $$5'\text{-Com-}N_{n1}\text{-}3' \hspace{5cm} I$$

    wherein Com is a sequence complementary to at least the 5'-end of an adapter fragment which is ligated to the 3'-end of a cleaved cDNA fragment, N is A, G, T, or C, the one primer having the general formula I where n1=0, and the second primer having the general formula I where $1 \leq n1 \leq 4$, said second primer being capable of priming amplification of any nucleotide sequence ligated in its 3'-end to the adapter fragment complementary in its 5'-end to Com.

2. A method according to claim 1, wherein the restriction endonuclease of step c) is a rare 4 base cutter, said rare 4 base cutter being chosen so as to cleave each complete cDNA into an average of about 3 fragments.

3. A method according to claim 1 or 2, wherein the rare 4 base cutter is selected form the group consisting of AciI, AluI, BfaI, BstUI, Csp6I, DpnI, DpnII, HaeIII, HhaI, HinP1I, HpaII, MboI, MnlI, MseI, MspI, NlaIII, RsaI, Sau3AI, TaiI, TaqI, and Tsp509I.

4. A method according to any of claims 1-3, wherein the termination fragment in step d) comprises or is a chemically modified nucleotide sequence, said modified nucleotide sequence being a dideoxynucleotide covalently attached to the 3'-end of the nucleotide strand.

5. A method according to any of claims 1-4, wherein the set of amplification primers having formula I wherein $1 \leq n1 \leq 4$ comprises all possible combinations and permutations of A, G, T and C in the group $N_{n1}$.

6. A method according to any of claims 1-5, wherein the set of amplification primers having n1=0 in formula I is labelled.

7. A method according to any of claims 1-6, wherein the set of amplification primers of formula I wherein $1 \leq n1 \leq 4$ has n1=3.

8. A method according to any of the preceding claims, which comprises the further step of separating amplified fragments obtained from the molecular amplification procedure of step f).

9. A method according to claim 8, wherein the separation is performed by gel electrophoresis or chromatography.

10. A method according to claim 8 or 9, which further comprises the step of identifying separated amplified fragments.

11. A method according to claim 10, wherein the identification is performed by visualization.

12. A method according to claim 11, wherein labelled nucleotides are visualized, the labelled nucleotides being part of a probe or of the amplified fragments.

13. A method according to claim 12, wherein the labelled nucleotides are the labelled nucleotides being part of the labelled primers as defined in claim 6.

14. A method according to claim 13, wherein the visualization is performed by incorporating radioactive or fluorescent alpha dNTP into the cDNA fragment during PCR, where N = A, C, T, U or G.

15. A method according to any of the preceding claims, wherein step b) is carried out under conditions which minimize the formation of mismatches between nucleotides in the first and second cDNA strands.

16. A method according to any of the preceding claims, wherein the mRNA is of eukaryotic, Archae or prokaryotic origin.

17. A method for determining the presence of an expression product in a cell or group of cells, the method comprising providing an RNA-containing sample from the cell or group of cells and subjecting the sample to the method according to any of claims 1-16, and thereafter performing a comparison of the thus identified amplified cDNA fragments with a database output, said database output comprising a computer-generated list of molecular weights of restriction DNA fragments of known sequences, said list being prepared by

- inputting and storing DNA sequence data in a database as virtual DNA sequences,
- subsequently simulating cleavage of the virtual DNA sequences with the at least one restriction nuclease and storing the resulting simulated cleavage products as virtually cleaved DNA fragments,
- simulating ligation to the virtually cleaved fragments of the at least two adapter fragments and storing the results as virtually ligated DNA fragments,
- for each individual combination of primers used in step f), grouping the virtually ligated DNA fragments susceptible to amplification by said combination of primers in the same group,
- determining, in each group, the absolute and/or relative molecular weight of each virtually ligated DNA fragment, and
- outputting the content of each group in the form of a list comprising the absolute and/or relative molecular weights of the virtually ligated fragments in the group.

18. A method according to claim 17, wherein the input DNA sequence data are linked to data relating to the genetic origin of the DNA sequence data and optionally to data relating to functional features relating to the genetic origin.

19. A method according to claim 17, wherein the output indication further comprises information about the genetic origin of the virtually ligated DNA fragment and optionally information about functional features associated with the genetic origin.

20. A method according to any of claims 17-19, wherein the comparison is performed by inputting the identified amplified cDNA fragments in a format which allows automated comparison with the database output, or, alternatively,

by outputting the database output in a format which allows for direct comparison between the separated amplified cDNA fragments and the database output.

21. A method for determining change in expression, compared to the expression in a reference cell or reference group of cells, of an expression product in a cell or group of cells which has been subjected to a first set of conditions influencing the expression pattern of said cell or group of cells, said reference cell or group of cells being subjected to a second set of conditions, the method comprising providing an RNA-containing sample from the cell or group of cells and subjecting the sample to the method according to any of claims 10-14 thereby obtaining data describing the amplified cDNA fragments derived from the sample, providing reference data describing amplified cDNA fragments derived from an RNA-containing reference sample from the reference cell or reference group of cells, the reference data being obtained by having previously subjected the reference sample to the method according to any of claims 10-14,
subsequently performing a comparison of the data and the reference data to identify those cDNA fragments which are expressed at different levels in the two data sets, and thereafter using the differentially expressed cDNA fragments to determine which expression products are subject to a change in expression level.

22. A method according to claim 21, wherein the data and reference data are selected from the group consisting of the apparent molecular weights of the amplified DNA fragments, the $M_r$ of the amplified DNA fragments, the absolute amount of the amplified DNA fragments, and the relative amounts of the amplified DNA fragments.

23. A method according to claim 21, wherein the reference data are extracted from a database containing the reference data defined in claim 22 and optionally further information relating to the genetic origin of each amplified cDNA fragment from the reference.

24. A method for diagnosing a disease in a subject, said disease being **characterized by** a deviating expression level of at least one expression product in at least one cell type, the method comprising providing an RNA-containing sample derived from the at least one cell type, subjecting the sample to the method according to any of claims 10-14 thereby obtaining data describing the amplified cDNA fragments derived from the sample, providing reference data describing amplified cDNA fragments derived from a RNA-containing reference sample derived from the same type of cell from a subject not suffering from the disease, the reference data being obtained by having previously subjected the reference sample to the method according to any of claims 10-14, and
subsequently performing a comparison of the data and the reference data with respect to those cDNA fragments which are known to be related to the disease, and assessing whether a significant difference in the data and reference data exists so as to establish whether the expression level of the expression product deviates or not.

25. A method according to claim 24, wherein the data and reference data are selected from the group consisting of the apparent molecular weights of the amplified DNA fragments, the $M_r$ of the amplified DNA fragments, the absolute amount of the amplified DNA fragments, and the relative amounts of the amplified DNA fragments.

26. A method according to claim 24, wherein the reference data are extracted from a database containing the reference data defined in claim 25 and optionally further information relating to the genetic origin of each amplified cDNA fragment from the reference.

27. A method of preparing a surface (chip) coated with cDNA fragments, the method comprising

    - subjecting an RNA-containing sample to the method of any of claims 8-14, and

    - transferring the separated amplified cDNA fragments to a chip surface adapted to stably bind the separated amplified cDNA fragments while maintaining the spatial relative distribution pattern thereof.

28. A method of preparing a surface (chip) coated with cDNA fragments, the method comprising

    - subjecting an RNA-containing sample to the method of any of claims 1-7,
    - separating, by electrophoresis, the thus obtained amplified cDNA fragments on a particular surface adapted to stably bind the separated amplified cDNA fragments while maintaining the relative distribution pattern after electrophoresis.

29. A method according to claim 28, wherein the electrophoresis is in the form of microelectrophoresis.

**30.** A method according to any of claims 27-29, wherein the transfer is accomplished by a electrophoretic blotting technique.

**31.** A method according to any of claims 27-29, wherein the transfer is accomplished by photo-activated organic or inorganic chemistry techniques.

**32.** A surface having cDNA stably bound thereto, said surface being obtainable by the method according to any of claims 27-31.

**33.** A method for the screening for genes within a family of genes, the method comprising providing a surface according to claim 32, wherein cDNA stably bound to the surface is hybridized under low stringency conditions to a detectably labelled nucleic acid which is a representative of a gene family, and subsequently analyzing fragments of the chip to which hybridization has occurred so as to determine whether such fragments are related to the same gene family.

**34.** A method for determining the difference in expression pattern between a first cell or type of cells and a second cell or type of cells, the method comprising providing samples of labelled RNA or cDNA from the first and second cells or cell types and subsequently contacting each of these samples with a surface according to claim 32, and subsequently detecting the amount and distribution of bound labelled RNA or cDNA from each sample.

**35.** A method for screening for interactions between a pre-selected protein and a polypeptide fragment, the method comprising preparing a sub-divided library of amplified cDNA fragments according to the method of any of claims 1-14, optionally adapting the terminals of the members of the library so as to facilitate insertion into a vector, inserting the fragments into vectors, transforming a population of suitable host cells with the vectors, culturing the host cells under conditions which enable expression of correctly inserted cDNA fragments by the host cell, and subsequently assaying polypeptide fragments encoded by the inserted cDNA fragments for interaction with the pre-selected protein.

**36.** A method according to claim 35, wherein assaying of the polypeptide fragments is performed by a two-hybrid technique, wherein the host cells are eukaryotic cells which are mated or transfected with nucleic acid material encoding the pre-selected protein, successful mating/transfection of the cell(s) resulting in a cell or cells wherein the interaction between the pre-selected protein and a polypeptide fragment gives rise to a detectable signal.

**37.** A method according to claim 36, wherein a fungus, such as a yeast cell, is used in the mating/transfection.

**38.** A method according to claim 36 or 37, wherein the detectable signal is provided by Green Fluorescent Protein.

**Patentansprüche**

**1.** Verfahren zum Herstellen einer normalisierten, unterteilten Bibliothek von amplifizierten cDNA-Fragmenten der codierenden Region von mRNA, die in einer Probe enthalten ist, wobei das Verfahren die Schritte umfasst:

   (a) Durchführen einer reversen Transkription mit der aus der Probe gewonnenen mRNA unter Verwendung mindestens eines cDNA-Primers, wobei cDNA-Fragmente des ersten Strangs erhalten werden,
   (b) Synthetisieren der cDNA des zweiten Strangs, die komplementär zu den cDNA-Fragmenten des ersten Strangs ist, unter Verwendung der DNA-Fragmente des ersten Strangs als Matrizen, wobei doppelsträngige cDNA-Fragmente erhalten werden,
   (c) Verdauen der doppelsträngen cDNA-Fragmente mit einer Restriktionsendonuclease, wobei die Restriktionsendonuclease überstehende Enden (klebrige Enden) gleicher Größe an den Endstellen der DNA nach dem Verdau erzeugt, wobei gespaltene cDNA-Fragmente erhalten werden,
   (d) Hinzufügen von mindestens zwei Adapterfragmenten, die bekannte Sequenzen umfassen, zu den gespaltenen cDNA-Fragmenten, welche in Schritt (c) erhalten wurden, wobei mindestens diese zwei Adapterfragmente in der Lage sind, spezifisch an die klebrigen Enden der doppelsträngigen cDNA zu binden, welche in Schritt (c) hergestellt wurde, wobei ein Adapterfragment an den Primer mit der Formel I aus Schritt (e) angelagert werden kann, das zweite Adapterfragment ein Terminationsfragment darstellt, welches eine Blockierung der Polymerisation der DNA in 5'->3' Richtung von diesem Terminationsfragment aus einführt und dieses Terminationsfragment nicht an einen Primer von mindestens zwei Sätzen von Primern aus Schritt (e) während eines molekularen Amplifikationsverfahrens angelagert werden kann, wobei die mindestens zwei Adapterfragmente mit den gespaltenen cDNA-Fragmenten, die in Schritt (c) erhalten wurden, ligiert werden, so dass ligierte cDNA-Fragmente erhalten werden,

(e) Aufteilen der ligierten cDNA-Fragmente, die in Schritt (d) erhalten wurden, in $4^{n1}$ Ansätze wobei $1 \leq n1 \leq 4$, und

(f) Verwenden jedes Ansatzes von ligierten cDNA-Fragmenten, die in Schritt (e) erhalten wurden, für ein molekulares Amplifikationsverfahren, um amplifizierte cDNA-Fragmente zu erhalten, unter Verwendung für ein Adapterfragment, welches in Schritt (d) verwendet wurde, eines Satzes von Amplifikationsprimem mit der allgemeinen Formel

$$5'\text{-Com-}N_{n1}\text{-}3'$$

wobei Com die komplementäre Sequenz zu mindestens dem 5'-Ende des einen Adapterfragments ist, welches mit dem 3'-Ende der gespaltenen cDNA-Fragmente ligiert ist, N entweder A, G, T oder C entspricht, der eine Primer die allgemeine Formel I hat, in der n1=0, und der zweite Primer die allgemeine Formel I hat, in der $1 \leq n1 \leq 4$ ist, und dieser zweite Primer in der Lage ist, die Amplifikation jeder Nucleotidsequenz, die mit ihrem 3'-Ende an das Adapterfragment ligiert ist, das in seinem 5'-Ende komplementär zu Com ist, einzuleiten.

2. Verfahren nach Anspruch 1, wobei die Restriktionsendonuclease aus Schritt (c) ein seltener 4 Basen-Schneider ist, welcher so gewählt wird, dass dieser jede cDNA in durchschnittlich etwa drei Fragmente spaltet.

3. Verfahren nach den Ansprüchen 1 oder 2, wobei der seltene 4 Basen-Schneider gewählt ist aus einer Gruppe bestehend aus AciI, AluI, BfaI, BstUI, Csp6I, DpnI, DpnII, HaeIII, HhaI, HinP1I, HpaII, MboI, MnlI, MseI, MspI, NlaIII, RsaI, Sau3AI, TaiI, TaqI und Tsp509I.

4. Verfahren nach einem der Ansprüche 1 bis 3, wobei das Terminationsfragment aus Schritt (d) eine chemisch modifizierte Nucleotidsequenz umfasst oder ist, wobei diese modifizierte Nucleotidsequenz ein Didesoxynucleotid ist, das mit dem 3'-Ende des Nucleotidstrangs kovalent verbunden ist.

5. Verfahren nach einem der Ansprüche 1 bis 4, wobei der Satz der Amplifikationsprimer mit der Formel I, in der $1 \leq n1 \leq 4$ ist, alle möglichen Kombinationen und Permutationen von A, G, T und C in der Gruppe $N_{n1}$ enthält.

6. Verfahren nach einem der Ansprüche 1 bis 5, wobei der Satz der Amplifikationsprimer mit n1=0 in der Formel I markiert ist.

7. Verfahren nach einem der Ansprüche 1 bis 6, wobei der Satz der Amplifikationsprimer der Formel I, in der $1 \leq n1 \leq 4$ ist, den Wert n1=3 hat.

8. Verfahren nach einem der vorangegangenen Ansprüche, umfassend als weiteren Schritt die Trennung der amplifizierten Fragmente, die durch das molekulare Amplifikationsverfahren aus Schritt (f) hergestellt wurden.

9. Verfahren nach Anspruch 8, wobei die Trennung durch Gelelektrophorese oder Chromatographie erfolgt.

10. Verfahren nach Anspruch 8 oder 9, femer umfassend den Schritt der Identifizierung der aufgetrennten, amplifizierten Fragmente.

11. Verfahren nach Anspruch 10, wobei die Identifizierung durch ein Sichtbarmachen erreicht wird.

12. Verfahren nach Anspruch 11, wobei die markierten Nucleotide sichtbar gemacht werden und die markierten Nucleotide Teil einer Sonde oder der amplifizierten Fragmente sind.

13. Verfahren nach Anspruch 12, wobei die markierten Nucleotide die markierten Nucleotide sind, die ein Teil der markierten Primer sind, welche in Anspruch 6 definiert sind.

14. Verfahren nach Anspruch 13, wobei das Sichtbarmachen erreicht wird durch das Einfügen radioaktiver oder fluoreszierender alpha-dNTP in die cDNA-Fragmente während der PCR, wobei N = A, C, T, U oder G.

15. Verfahren nach einem der vorangegangenen Ansprüche, wobei Schritt (b) unter Bedingungen durchgeführt wird, welche die Bildung von Fehlpaarungen zwischen Nucleotiden des ersten und zweiten cDNA-Strangs minimieren.

16. Verfahren nach einem der vorangegangenen Ansprüche, wobei die mRNA eukaryontischen, archebakterischen oder prokaryontischen Ursprungs ist.

17. Verfahren zum Nachweis der Anwesenheit eines Expressionsprodukts in einer Zelle oder Gruppe von Zellen, umfassend die Bereitstellung einer RNA enthaltenden Probe einer Zelle oder Gruppe von Zellen und Verwenden dieser Probe in einem Verfahren nach einem der Ansprüche 1 bis 16 und einem anschließenden Vergleich der auf diese Weise identifizierten, amplifizierten cDNA-Fragmente mit dem Inhalt einer Datenbank, wobei der Inhalt dieser Datenbank eine computergenerierte Liste von Molekulargewichten von Restriktions-DNA-Fragmenten von bekannten Sequenzen umfasst, wobei die Liste erstellt wurde durch

- Eingeben und Speichern von DNA-Sequenzdaten in eine Datenbank als virtuelle DNA-Sequenzen,
- anschließendes Simulieren einer Spaltung der virtuellen DNA-Sequenzen mit mindestens einer Restriktionsendonuclease und Speichern der resultierenden simulierten Spaltprodukte als virtuelle, gespaltene DNA-Fragmente,
- Simulieren einer Ligierung der virtuell gespaltenen Fragmente mit mindestens zwei Adapterfragmenten und Speichern der resultierenden, virtuell ligierten DNA-Fragmente,
- für jede einzelne Kombination von Primern, die in Schritt (f) verwendet werden, Gruppieren der virtuell verknüpften DNA-Fragmente, die für die Amplifikation mit der Kombination von Primem empfänglich sind, zu einer Gruppe,
- Bestimmen des absoluten und/oder relativen Molekulargewichts jedes virtuell ligierten DNA-Fragments in jeder Gruppe, und
- Ausgabe des Inhalts jeder Gruppe in Form einer Liste umfassend die absoluten und/oder relativen Molekulargewichte der virtuell ligierten Fragmente in dieser Gruppe.

18. Verfahren nach Anspruch 17, wobei die eingegebenen DNA-Sequenzdaten verknüpft sind mit Daten, die sich auf den genetischen Ursprung der DNA-Sequenzdaten beziehen, und gegebenenfalls mit Daten, die sich auf funktionelle Merkmale beziehen, die sich auf den genetischen Ursprung beziehen.

19. Verfahren nach Anspruch 17, wobei die ausgegebenen Daten ferner Informationen über den genetischen Ursprung des virtuell ligierten DNA-Fragments und gegebenenfalls Informationen über funktionelle Merkmale liefern, die mit dem genetischen Ursprung verbunden sind.

20. Verfahren nach einem der Ansprüche 17 bis 19, wobei der Vergleich durchgeführt wird durch die Eingabe der identifizierten amplifizierten DNA-Fragmente in einem Format, welches den automatischen Vergleich mit der Datenbankausgabe ermöglicht, oder, alternativ, durch Ausgabe der Datenbankausgabe in einem Format, welches den direkten Vergleich zwischen den getrennten amplifizierten cDNA-Fragmenten und der Datenbankausgabe erlaubt.

21. Verfahren zur Bestimmung von Änderungen in der Expression, im Vergleich zur Expression in einer Referenzzelle oder einer Referenzgruppe von Zellen, eines Expressionsprodukts in einer Zelle oder Gruppe von Zellen, welche unter einer ersten Reihe von Bedingungen zur Beeinflussung des Expressionsmusters dieser Zelle oder Gruppe von Zellen inkubiert wird, wobei die Referenzzelle oder die Referenzgruppe von Zellen einer zweiten Reihe von Bedingungen unterworfen wird, das Verfahren umfassend die Bereitstellung einer RNA enthaltenden Probe von dieser Zelle oder Gruppe von Zellen und Verwendung dieser Probe in Verfahren nach einem der Ansprüche 10 bis 14, wobei Daten erhalten werden, welche die amplifizierten cDNA-Fragmente, die aus der Probe erhalten werden, beschreiben, Bereitstellung von Referenzdaten, welche die amplifizierten cDNA-Fragmente, die aus RNA enthaltenden Referenzproben von der Referenzzelle oder der Referenzgruppe von Zellen erhalten wurden, beschreiben, wobei diese Referenzdaten erhalten wurden durch eine vorangegangene Verwendung der Referenzproben in dem Verfahren nach einem der Ansprüche 10 bis 14, anschließende Durchführung eines Vergleichs der Daten und der Referenzdaten zur Identifizierung solcher cDNA-Fragmente, welche in unterschiedlichem Grad in diesen beiden Datensätzen exprimiert werden, und anschließend Verwendung der unterschiedlich exprimierten cDNA-Fragmente, um zu bestimmen, bei welchen Expressionsprodukten unterschiedliche Expressionsgrade auftreten.

22. Verfahren nach Anspruch 21, wobei die Daten und Referenzdaten gewählt sind aus der Gruppe, bestehend aus den scheinbaren Molekulargewichten der amplifizierten DNA-Fragmente, dem $M_r$ dieser amplifizierten DNA-Frag-

mente, der absoluten Menge der amplifizierten DNA-Fragmente und der relativen Mengen der amplifizierten DNA-Fragmente.

23. Verfahren nach Anspruch 21, wobei die Referenzdaten aus einer Datenbank stammen, welche nach Anspruch 22 definierte Referenzdaten umfasst und gegebenenfalls weitere Informationen, die sich auf den genetischen Ursprung jedes amplifizierten cDNA-Fragments dieser Referenz beziehen.

24. Verfahren zur Diagnose einer Erkrankung in einem Individuum, wobei die Erkrankung durch einen abweichenden Expressionsgrad von mindestens einem Expressionsprodukt in mindestens einem Zelltyp **gekennzeichnet** ist, umfassend die Bereitstellung einer RNA enthaltenden Probe, die aus mindestens einem Zelltyp gewonnen wird, Verwendung dieser Probe in einem Verfahren nach einem der Ansprüche 10 bis 14, wobei die dabei erhaltenen Daten die amplifizierten cDNA-Fragmente dieser Probe beschreiben, das Bereitstellen von Referenzdaten, welche die amplifizierten cDNA-Fragmente beschreiben, welche aus einer RNA enthaltenden Referenzprobe stammen, welche aus dem gleichen Zelltyp von einem Individuum, welches nicht unter dieser Erkrankung leidet, gewonnen wurde, wobei diese Referenzdaten erhalten wurden durch eine vorangegangene Verwendung der Referenzprobe in einem Verfahren nach einem der Ansprüche 10 bis 14, und anschließendem Vergleich der erhaltenen Daten mit den Referenzdaten im Hinblick auf die cDNA-Fragmente, von welchen bekannt ist, dass diese mit der Erkrankung in Verbindung stehen, und Abschätzen, ob eine signifikante Differenz zwischen den Daten und Referenzdaten besteht, um festzustellen, ob der Expressionsgrad eines Expressionsprodukts abweicht oder nicht.

25. Verfahren nach Anspruch 24, wobei die Daten und die Referenzdaten ausgewählt sind aus der Gruppe bestehend aus den scheinbaren Molekulargewichten der amplifizierten DNA-Fragmente, dem $M_r$ der amplifizierten DNA-Fragmente, der absoluten Menge der amplifizierten DNA-Fragmente und den relativen Mengen der amplifizierten DNA-Fragmente.

26. Verfahren nach Anspruch 24, wobei die Referenzdaten aus einer Datenbank stammen, welche die in Anspruch 25 definierten Referenzdaten umfasst und gegebenenfalls weitere Informationen, die sich auf den genetischen Ursprung jedes amplifizierten cDNA-Fragments dieser Referenz beziehen.

27. Verfahren zur Herstellung einer mit cDNA-Fragmenten beschichteten Oberfläche (Chip), umfassend

- Verwendung einer RNA enthaltenden Probe in einem Verfahren nach einem der Ansprüche 8 bis 14, und
- Übertragen der getrennten amplifizierten cDNA-Fragmente auf eine Chip-Oberfläche, welche für eine stabile Bindung der getrennten amplifizierten cDNA-Fragmente angepaßt ist, wobei das relative räumliche Verteilungsmuster aufrechterhalten wird.

28. Verfahren zur Herstellung einer mit cDNA-Fragmenten beschichteten Oberfläche (Chip), umfassend

- Verwendung einer RNA enthaltenden Probe in einem Verfahren nach einem der Ansprüche 1 bis 7,
- Auftrennung der so erhaltenen amplifizierten cDNA-Fragmente durch Elektrophorese auf einer spezieller Oberfläche, welche für eine stabile Bindung der getrennten amplifizierten cDNA-Fragmente angepasst ist, wobei das relative Verteilungsmuster nach der Elektrophorese erhalten bleibt.

29. Verfahren nach Anspruch 28, wobei die Elektrophorese in Form einer Mikroelektrophorese durchgeführt wird.

30. Verfahren nach einem der Ansprüche 27 bis 29, wobei der Transfer durch eine elektrophoretische Blot-Technik gewährleistet wird.

31. Verfahren nach einem der Ansprüche 27 bis 29, wobei der Transfer durch fotoaktive, organische oder anorganische chemische Techniken gewährleistet wird.

32. Oberfläche, an welche cDNA stabil gebunden ist, wobei die Oberfläche erhältlich ist durch das Verfahren nach einem der Ansprüche 27 bis 31.

33. Verfahren zur Durchmusterung von Genen innerhalb einer Familie von Genen, umfassend die Bereitstellung einer Oberfläche nach Anspruch 32, wobei die an die Oberfläche stabil gebundene cDNA unter wenig stringenten Bedingungen hybridisiert wird mit einer nachweisbar markierten Nucleinsäure, die kennzeichnend für eine Familie von Genen ist, und anschließender Analyse der Fragmente auf dem Chip, für die eine Hybridisierung beobachtet wurde, und auf diesem Wege Bestimmen, ob diese Fragmente zur gleichen Genfamilie gehören.

34. Verfahren zur Bestimmung einer Differenz im Expressionsmuster zwischen einer ersten Zelle oder einem Zelltyp und einer zweiten Zelle oder einem Zelltyp, umfassend die Bereitstellung von markierten RNA- oder cDNA-Proben der ersten und der zweiten Zelle oder des Zelltyps, anschließend das Inkontaktbringen jede dieser Proben mit einer Oberfläche nach Anspruch 32 und anschließender Nachweis der Menge und Verteilung von gebundener, markierter RNA oder cDNA jeder Probe.

35. Verfahren zur Durchmusterung von Wechselwirkungen zwischen einem vorher ausgewählten Protein und einem Polypeptidfragment, umfassend die Bereitstellung einer unterteilten Bibliothek von amplifizierten cDNA-Fragmenten gemäß einem Verfahren nach einem der Ansprüche 1 bis 14, gegebenenfalls Anpassen der Enden der Mitglieder dieser Bibliothek, um das Einfügen in einen Vektor zu erleichtern, das Einfügen der Fragmente in Vektoren, das Transformieren einer Population geeigneter Wirtszellen mit den Vektoren, Züchten der Wirtszellen unter Bedingungen, welche die Expression von korrekt eingefügten cDNA-Fragmenten in der Wirtszelle ermöglichen und anschließende Analyse der Polypeptidfragmente, die von den eingefügten cDNA-Fragmenten codiert werden, bezüglich ihrer Wechselwirkung mit dem vorher ausgewählten Protein.

36. Verfahren nach Anspruch 35, wobei die Polypeptidfragmente mit einer Zweihybrid-Technik analysiert werden, wobei die Wirtszellen eukaryontische Zellen sind, welche gepaart oder mit Nucleinsäurematerial transfiziert werden, das das vorher ausgewählte Protein codiert, wobei die erfolgreiche Paarung/Transfektion dieser Zelle(n) zur Bereitstellung einer Zelle oder von Zellen führt, in welchen die Wechselwirkung zwischen dem vorher ausgewählten Protein und einem Polypeptidfragment ein nachweisbares Signal auslöst.

37. Verfahren nach Anspruch 36, wobei ein Pilz, wie eine Hefezelle, für die Paarung/Transfektion verwendet wird.

38. Verfahren nach einem der Ansprüche 36 oder 37, wobei das nachweisbare Signal durch das grün fluoreszierende Protein bereitgestellt wird.

## Revendications

1. Méthode de préparation d'une bibliothèque normalisée et sub-divisée de fragments d'ADNc amplifiés à partir de la région codante de l'ARNm contenu dans un échantillon, la méthode comprenant les étapes de

a) transcription inverse de l'ARNm obtenu à partir de l'échantillon, en utilisant au moins une amorce d'ADNc, obtenant ainsi des fragments d'ADNc simple brin,
b) synthèse du deuxième brin d'ADNc complémentaire des fragments du premier brin d'ADNc en utilisant les fragments du premier brin d'ADN comme matrices, obtenant ainsi des fragments d'ADNc double brin,
c) digestion des fragments d'ADNc double brin avec une endonucléase de restriction, ladite endonucléase de restriction générant des extrémités saillantes (extrémités cohésives) de taille similaire aux extrémités de l'ADN après la digestion, fournissant ainsi des fragments d'ADNc coupé,
d) addition d'au moins deux fragments adaptateurs contenant des séquences connues aux fragments d'ADNc coupé obtenus à l'étape c), lesdits au moins deux fragments adaptateurs étant capables de se lier spécifiquement aux extrémités cohésives de l'ADNc double brin produit à l'étape c), le premier fragment adaptateur étant capable de s'hybrider à l'amorce ayant la formule I dans l'étape c), 1c second fragment adaptateur étant un fragment de terminaison introduisant un bloc contre la polymérisation de l'ADN dans la direction $5' \rightarrow 3'$ à partir dudit fragment de terminaison et ledit fragment de terminaison étant incapable de s'hybrider à une quelconque amorce des jeux d'au moins deux amorces à l'étape e) pendant la procédure d'amplification moléculaire, les au moins deux fragments adaptateurs étant liés aux fragments d'ADNc coupés obtenus à l'étape c), de manière à obtenir des fragments d'ADNc liés,
e) sub-division des fragments d'ADNc liés obtenus à l'étape d) en $4^{n1}$ groupe dans lesquels $1 \leq n1 \leq 4$, et
f) soumission de chaque groupe de fragments d'ADNc ligaturés obtenus à l'étape e) à une procédure d'amplification moléculaire de manière à obtenir des fragments d'ADNC amplifiés, dans laquelle est utilisé pour un fragment adaptateur utilisé dans l'étape d), un jeu d'amorces d'amplification ayant la formule générale 5'-Com-$N_{n1}$-3'

dans laquelle Com est une séquence complémentaire à au moins l'extrémité 5' d'un fragment adaptateur qui est ligaturé à l'extrémité 3' d'un fragment d'ADNc coupé, N est A, G, T, ou C, la première amorce ayant la formule générale I dans laquelle n1=0, et la deuxième amorce ayant la formule générale I dans laquelle 1≤n1≤4, ladite seconde amorce étant capable d'initier l'amplification de toute séquence de nucléotides ligaturée à son extrémité 3' au fragment adaptateur complémentaire à son extrémité 5' à Com.

**2.** Méthode selon la revendication 1, dans laquelle l'endonucléase de restriction de l'étape c) est un enzyme de restriction à séquence de reconnaissance de 4 bases coupant rarement, ledit enzyme de restriction à séquence de reconnaissance de 4 bases coupant rarement étant choisi de manière à couper chaque ADNc complet en environ 3 fragments en moyenne.

**3.** Méthode selon la revendication 1 ou 2, dans laquelle l'enzyme de restriction à séquence de reconnaissance de 4 bases coupant rarement est choisi parmi le groupe constitué par Acil, AluI, BfaI, BstUI, Csp6I, DpnI, DpnII, HaeIII, HhaI, HinP1I, HpaII, MboI, MnlI, MseI, MspI, NlaIII, RsaI, Sau3AI, Tail, TaqI, et Tsp5091.

**4.** Méthode selon l'une quelconque des revendications 1 à 3, dans laquelle le fragment de terminaison de l'étape d) comprend une ou est une séquence nucléotidique chimiquement modifiée, ladite séquence nucléotidique modifiée étant un didésoxynucléotide attaché de manière covalente à l'extrémité 3' du brin de nucléotide,

**5.** Méthode selon l'une des revendications 1 à 4, dans laquelle le jeu d'amorce d'amplification ayant la formule 1 dans laquelle 1≤n1≤4 comprend toutes les combinaisons et permutations possibles de A, G, T et C dans le groupe $N_{n1}$.

**6.** Méthode selon l'une quelconque des revendications 1 à 5, dans laquelle le jeu d'amorces d'amplification ayant le chiffre n1 = 0 dans la formule I est marqué.

**7.** Méthode selon l'une quelconque des revendications 1 à 6, dans laquelle le jeu d'amorces d'amplification de la formule I dans laquelle 1≤n1≤4 a le chiffre n1 = 3.

**8.** Méthode selon l'une quelconque des revendications précédentes, qui comprend l'étape supplémentaire de séparation des fragments amplifiés obtenus par la procédure d'amplification moléculaire de l'étape f).

**9.** Méthode selon la revendication 8, dans laquelle la séparation est effectuée par électrophorèse sur gel ou chromatographie.

**10.** Méthode selon la revendication 8 ou 9, comprenant en outre l'étape d'identification des fragments amplifiés séparés.

**11.** Méthode selon la revendication 10, dans laquelle l'identification est effectuée par visualisation.

**12.** Méthode selon la revendication 11, dans laquelle les nucléotides marqués sont visualisés, les nucléotides marqués étant une partie de la sonde ou des fragments amplifiés.

**13.** Méthode selon la revendication 12, dans laquelle les nucléotides marqués sont les nucléotides marqués constituant une partie des amorces marquées telles que celles définies dans la revendication 6.

**14.** Méthode selon la revendication 13, dans laquelle la visualisation est effectuée en incorporant un alpha dNTP radioactif ou fluorescent dans le fragment d'ADNc pendant la PCR, dans laquelle N = A, C, T, U ou G.

**15.** Méthode selon l'une quelconque des revendications précédentes, dans laquelle l'étape b) est effectuée dans des conditions qui minimisent la formation de mésappariements entre les nucléotides dans les premier et second brin d'ADNc.

**16.** Méthode selon l'une quelconque des revendications précédentes, dans laquelle l'ARNm est d'origine eucaryotique, archae ou procaryotique.

**17.** Méthode de détermination de la présence d'un produit d'expression dans une cellule ou un groupe de cellules, la méthode consistant à fournir un échantillon contenant un ARN à partir de la cellule ou du groupe de cellules et à soumettre l'échantillon à la méthode selon l'une quelconque des revendications 1 à 16, et à effectuer ensuite une comparaison de fragments d'ADNc amplifiés ainsi identifiés avec une sortie d'une base de données, ladite sortie d'une base de données comprenant une liste établie par ordinateur de poids moléculaires de fragments de restriction d'ADN de séquences connues, ladite liste étant préparée par

- introduction et stockage des données de séquence d'ADN dans une base de données en tant que séquences d'ADN virtuelles,
- stimulation subséquente de la coupure des séquences virtuelles d'ADN avec au moins une nucléase de restriction et stockage des produits de coupure simulée résultants comme des fragments d'ADN virtuellement coupés,
- stimulation de la ligation aux fragments virtuellement coupés d'au moins deux fragments adaptateurs et stockage des résultats comme des fragments d'ADN virtuellement liés,
- pour chaque combinaison individuelle d'amorces utilisée dans l'étape f), grouper les fragments d'ADN liés virtuellement susceptibles d'amplification par ladite combinaison d'amorces dans le même groupe,
- détermination dans chaque groupe, du poids moléculaire absolu et/ou relatif de chaque fragment d'ADN virtuellement ligaturé, et
- sortie du contenu de chaque groupe sous la forme d'une liste comprenant les poids moléculaires absolus et/ou relatifs des fragments virtuellement ligaturés dans le groupe.

**18.** Méthode selon la revendication 17, dans laquelle les données de séquence d'ADN introduites sont liées aux données concernant l'origine génétique des données de séquence d'ADN et facultativement aux données concernant les caractéristiques fonctionnelles concernant l'origine génétique.

**19.** Méthode selon la revendication 17, dans laquelle l'indication de sortie comprend en outre des informations sur l'origine génétique du fragment d'ADN virtuellement ligaturé et facultativement les informations concernant les caractéristiques fonctionnelles associées à l'origine génétique.

**20.** Méthode selon l'une quelconque des revendications 17 à 19, dans laquelle la comparaison est effectuée en introduisant les fragments d'ADNc amplifiés identifiés sous un format qui permet la comparaison automatisée avec la sortie de la base de données, ou, alternativement, en extrayant la sortie de la base de données sous un format qui permet une comparaison directe entre les fragments d'ADNc amplifiés séparés et la sortie de base de données.

**21.** Méthode de détermination du changement d'expression, comparé à l'expression dans une cellule de référence ou dans un groupe de cellules de référence d'un produit d'expression dans une cellule ou un groupe de cellules qui a été soumis à un premier ensemble de conditions influençant le profil d'expression de ladite ou cellule ou dudit groupe de cellules, ladite cellule de référence ou ledit groupe de cellules étant soumis à un deuxième ensemble de conditions, la méthode comprenant la fourniture d'un échantillon contenant un ARN de la cellule ou du groupe de cellules et la soumission de l'échantillon à la méthode selon l'une quelconque des revendications 10 à 14 obtenant ainsi des données décrivant les fragments d'ADNc amplifiés obtenus à partir de l'échantillon, fournissant les données de référence décrivant les fragments d'ADNc amplifiés obtenus à partir d'un échantillon de référence contenant de l'ARN de la cellule de référence ou du groupe de cellules de référence, les données de référence étant obtenues en ayant préalablement soumis l'échantillon de référence à la méthode selon l'une quelconque des revendications 10 à 14, effectuer ensuite une comparaison des données et des données de référence pour identifier les fragments d'ADNc qui sont exprimés à différents niveaux dans les deux séries de données, et utiliser ensuite les fragments d'ADN exprimés différentiellement pour déterminer quels produits d'expression présentent un changement au niveau du taux d'expression.

**22.** Méthode selon la revendication 21, dans laquelle les données et les données de référence sont choisies parmi le groupe constitué par les poids moléculaires apparents des fragments d'ADN amplifiés, le Rm des fragments d'ADN amplifiés, la quantité absolue de fragments d'ADN amplifiés, et les quantités relatives des fragments d'ADN amplifiés.

**23.** Méthode selon la revendication 21, dans laquelle les données de référence sont sorties d'une base de données contenant les données de référence définies dans la revendication 22 et facultativement d'autres informations concernant l'origine génétique de chaque fragment d'ADNc amplifié à partir de la référence.

**24.** Méthode de diagnostic d'une maladie chez un sujet, ladite maladie étant **caractérisée par** un taux d'expression modifié d'au moins un produit d'expression dans au moins un type de cellule, la méthode comprenant la fourniture d'un échantillon contenant un ARN obtenu à partir d'au moins un type cellulaire, la soumission de l'échantillon à la méthode selon l'une quelconque des revendications 10 à 14, obtenant ainsi des données décrivant les fragments d'ADNc amplifiés obtenus à partir de l'échantillon, la fourniture des données de référence décrivant les fragments d'ADNc amplifiés obtenus à partir d'un échantillon de référence contenant de l'ARN obtenu à partir du même type de cellule d'un sujet ne souffrant pas de la maladie, les données de référenee étant obtenues en ayant préalablement soumis l'échantillon de référence à la méthode selon l'une quelconque des revendications 10 à 14, et la réalisation subséquente d'une comparaison des données et des données de référence par rapport aux fragments d'ADNc connus comme étant reliés à la maladie, et l'évaluation de l'existence d'une différence significative entre les données et les données de référence de manière à établir si le taux d'expression du produit d'expiession est différent ou non.

**25.** Méthode selon la revendication 24, dans laquelle les données et les données de référence sont choisies parmi le groupe constitué par les poids moléculaires apparents des fragments d'ADN amplifiés, le Rm des fragments d'ADN amplifiés, la quantité absolue de fragments d'ADN amplifiés et les quantités relatives des fragments d'ADN amplifiés.

**26.** Méthode selon la revendication 24, dans laquelle les données de référence sont extraites d'une base de données contenant les données de référence définies dans la revendication 25 et optionnellement d'autres informations concernant l'origine génétique de chaque fragment d'ADN amplifié de la référence.

**27.** Méthode de préparation d'une surface (puce) revêtue de fragments d'ADNc, la méthode consistant à

- soumettre un échantillon contenant de l'ARN à la méthode selon l'une quelconque des revendications 8 à 14, et
- transférer les fragments d'ADNc amplifiés séparés sur une surface de puce adaptée pour lier de maniére stable les fragments d'ADNc amplifiés séparés tout en maintenant le profil de distribution spatiale relative de ceux-ci.

**28.** Méthode de préparation d'une surface (puce) revêtue de fragments d'ADNc, la méthode consistant à

- soumettre un échantillon contenant de l'ARN à la méthode selon l'une quelconque des revendications 1 à 7,
- séparer, par électrophorèse, les fragments d'ADNc amplifiés ainsi obtenus sur une surface particulière adaptée pour lier de maniére stable les fragments d'ADNc amplifiés séparés tout en maintenant le profil de distribution spatiale relative après l'électrophorèse.

**29.** Méthode selon la revendication 28, dans laquelle l'électrophorèse est sous forme de microélectrophorèse.

**30.** Méthode selon l'une quelconque des revendications 27 à 29, dans laquelle le transfert est effectué par une technique de blotting électrophorétique.

**31.** Méthode selon l'une quelconque des revendications 27 à 29, dans laquelle le transfert est effectué par des techniques de chimie organique ou inorganique photoactivécs.

**32.** Surface comportant un ADNc y étant lié de manière stable, ladite surface étant obtenue par la méthodes selon l'une quelconque des revendications 27 à 31.

**33.** Méthode de criblage de gènes parmi une famille de gènes, la méthode consistant à fournir une surface selon la revendication 32, dans laquelle l'ADNc lié de manière stable à la surface est hybridé dans des conditions faiblement astringentes à un acide nucléique marqué de manière détectable qui est représentatif d'une famille de gène, et à analyser subséquemment les fragments de puce sur lesquels l'hybridation s'est effectuée de manière à déterminer si ces fragments sont reliés à la même famille de gènes.

**34.** Méthode de détermination de la différence de profil d'expression entre une première cellule ou un premier type de cellules et une seconde cellule ou un second type de cellules, la méthode consistant à fournir des échantillons d'ARN ou d'ADNc marqués des première cellules ou des premiers types cellulaires et des secondes cellules ou des seconds types cellulaires et à mettre en contact subséquemment chacun de ces échantillons avec une surface selon la revendication 32, et à détecter subséquemment la quantité et la distribution de l'ARN ou de l'ADNc marqués liés de chaque échantillon.

**35.** Méthode de criblage des interactions entre une protéine présélectionnée et un fragment de polypeptide, la méthode consistant à préparer une bibliothèque sub-divisée de fragments d'ADNc amplifiés avec la méthode selon l'une quelconque des revendications 1 à 14, à adapter facultativement les extrémités des membres de la bibliothèque de manière à faciliter l'insertion dans un vecteur, insérer les fragments dans des vecteurs, transformer une population de cellules hôtes appropriées avec les vecteurs, cultiver les cellules hôtes dans des conditions qui permettent l'expression de fragments d'ADNc correctement insérés par la cellule hôte, et subséquemment essayer l'interaction des fragments de polypeptides codés par les fragments d'ADNc insérés avec la protéine présélectionnée.

36. Méthode selon la revendication 35, dans laquelle l'essai des fragments de polypeptides est effectué par une technique deux-hybrides, dans laquelle les cellules hôtes sont des cellules eucaryotiques qui sont accouplées ou transfectées avec un matériel acide nucléique codant la protéine présélectionnée, l'accouplement/transfection réussi(e)s de la (es) cellule(s) donnant une ou des cellule(s) dans lesquelles l'interaction entre la protéine présélectionnée et un fragment de polypeptide donne naissance à un signal détectable.

37. Méthodes selon la revendication 36, dans laquelle un champignon, tel qu'une cellule de levure, est utilisé dans l'accouplement/transfection.

38. Méthode selon la revendication 36 ou 37, dans laquelle le signal détectable est fourni par une protéine à fluorescence verte.

# Fig. 1  Basis of DODET

Cell A          Cell B

↓               ↓

Uninduced mRNA  Induced mRNA

↓               ↓

First and second strand cDNA synthesis

Restriction endonuclease digestion of cDNA

5'————————————————————————————— AAAAAAAAAAAA 3'
3'————————————————————————————— TTTTTTTTTTTT 5'

  b     b     b    a      b      b

Ligation of specific anchores

Continue next page

# ● *Fig. 1 Basis of DODET (continue)*

Pre-amplification

Second PCR amplification

Electrophoretic separation

Induced RNA

Cell A    Cell B

Q = Radioactive or fluorescent label

N = A or C or G or T

**● *Fig. 2 Anchor and PCR primer design***

Taq I site                               Bcl I site
↓                                        ↓

5' NNNNTCGANNNNNNNNNNNNNNNNNNNNTGATCANNN 3'
3' NNNNAGCTNNNNNNNNNNNNNNNNNNNNNACTAGTNNN 5'

Incubating with endonucleases
TaqI and Bcl I

CGANNNNNNNNNNNNNNNNNNNNNT
TNNNNNNNNNNNNNNNNNNNNNACTAG

Ligations of anchors

Anchor and endonuclease sequence                    Anchor and endonuclease sequence

NNNNNNNNNNNNNNNNNNNNV CGANNNNNNNNNNNNNNNNNNNNNNNTGATCBNNNNNNNNNNNNNNNN
NNNNNNNNNNNNNNNNNNNNBGC TNNNNNNNNNNNNNNNNNNNNNNNNACTAGVNNNNNNNNNNNNNNNN

**Continue next page**

○ *Fig. 2 Anchor and PCR primer design (continue)*

5' NNNNNNNNNNNNNNNNNNNVCGANNNNNNNNNNNNNNNNNNNNNTGATCBNNNNNNNNNNNNNNNNNNNN 3'
3' NNNNNNNNNNNNNNNNNNNBGCTNNNNNNNNNNNNNNNNNNNNNACTAGVNNNNNNNNNNNNNNNNNNNN 5'

Second PCR amplification

5'NNNNNNNNNNNNNNNNNNNVCGAT 3'
5'NNNNNNNNNNNNNNNNNNNVCGAG 3'
5'NNNNNNNNNNNNNNNNNNNVCGAC 3'
5'NNNNNNNNNNNNNNNNNNNVCGAA 3'

NNNNNNNNNNNNNNNNNNNVCGANNNNNNNNNNNNNNNNNNNTGATCBNNNNNNNNNNNNNNNN
NNNNNNNNNNNNNNNNNNNBGCTNNNNNNNNNNNNNNNNNNNACTAGVNNNNNNNNNNNNNNNN

3' AACTAGVNNNNNNNNNNNNNNNNNN—○5'
3' CACTAGVNNNNNNNNNNNNNNNNNN—○5'
3' GACTAGVNNNNNNNNNNNNNNNNNN—○5'
3' TACTAGVNNNNNNNNNNNNNNNNNN—○5'

N = A or C or G or T

—○ = Radioactive or
fluorescent label

Electrophoretic separation

# Fig. 3

Lane
1-4 5-8 9-12 13-16 17-20 21-24 25-28 29-32 33-36 37-40 41-44 45-48

## Fig. 4a

a b c d

## Fig. 4b

a b c d

## Fig. 4c

a b c d

## Fig. 4d

a b c d

# Fig. 5

# Fig. 6

# Fig. 7

mRNA

↓ cDNA synthesis

AAAAA
TTTTT

↓ Digestion with one endonuclease

Primer annealing
region

$$\left( \overline{\quad\quad dd\ \underline{\quad}_{PO_4} } \right)$$

labelled primer

Adapter-
ligation

dd

L → →
dd          dd
← L

+

L → →
dd          NNN ←

+

→ NNN
NNN ←

↓                    ↓ PCR Amplification                    ↓

No annealing and
amplification

64 reactions to be analysed
by e.g. gel-electrophoresis

No display of rare
amplified fragments
p = 1/64 x 1/64.

1    2    3  + ----- +  64

# Fig. 8

# displayARRAY principle

# Fig. 9

mRNA

cDNA synthesis

5' ———————————————————— AAAAA 3'
3' ———————————————————— VTTTTT
                                    con$_1$ 5'

ds cDNA synthesis

3' ———————————————————— VTTTTT—con$_1$ 5'
       NNNN ———————————————————— 3'
5' con$_2$

First PCR amplification
Primer set  con$_1$ + con$_2$

Second PCR amplification
Primer set  con$_1$ +  { con$_2$ AAA
                         con$_2$ AAC
                         ...
                         ...
                         con$_2$ GGG

64 reactions to be analysed
by e.g. gel-electrophoresis